(19) Europäisches Patentamt
European Patent Office
Office européen des brevets

(11) EP 1 027 316 B1

(12) **EUROPEAN PATENT SPECIFICATION**

(45) Date of publication and mention of the grant of the patent:
**21.05.2003 Bulletin 2003/21**

(21) Application number: **97910139.1**

(22) Date of filing: **10.10.1997**

(51) Int Cl.[7]: **C07C 43/20**, C07C 41/16 // C07C253/30, C07D307/79, C07D311/58, C07D313/08

(86) International application number:
**PCT/US97/18719**

(87) International publication number:
**WO 98/015515 (16.04.1998 Gazette 1998/15)**

# (54) SYNTHESIS OF ARYL ETHERS, METHODS AND REAGENTS RELATED THERETO

HERSTELLUNG VON ARYLETHERN SOWIE DAMIT VERBUNDENE REAGENTIEN UND VERFAHREN

SYNTHESE D'ETHERS D'ARYLE, PROCEDES ET REACTIFS AFFERENTS

(84) Designated Contracting States:
**AT BE CH DE DK ES FI FR GB GR IE IT LI LU MC NL PT SE**

(30) Priority: **10.10.1996 US 728449**

(43) Date of publication of application:
**16.08.2000 Bulletin 2000/33**

(60) Divisional application:
**02012164.6 / 1 245 553**
**02012284.2 / 1 254 884**

(73) Proprietor: **MASSACHUSETTS INSTITUTE OF TECHNOLOGY**
**Cambridge, MA 02139 (US)**

(72) Inventors:
- **BUCHWALD, Stephen, L.**
  **Newton, MA 02158 (US)**
- **WOLFE, John, P.**
  **Brighton, MA 02135 (US)**
- **PALUCKI, Michael**
  **Somerville, MA 02144 (US)**

(74) Representative: **Horner, Martin Grenville et al**
**Cruikshank & Fairweather**
**19 Royal Exchange Square**
**Glasgow G1 3AE Scotland (GB)**

(56) References cited:
- **R. B. BATES ET AL: "High-yield benzyne synthesis of diaryl ethers" JOURNAL OF ORGANIC CHEMISTRY, vol. 47, no. 22, 22 October 1982, EASTON US, pages 4374-4376, XP002053317**
- **R. CRAMER ET AL: "Nickel-catalyzed displacement reactions of aryl halides" JOURNAL OF ORGANIC CHEMISTRY, vol. 40, no. 16, 8 August 1975, EASTON US, pages 2267-2273, XP002053318 cited in the application**
- **H-J. CRISTAU ET EL: "Arylation of hard heteroatomic nucleophiles using bromoarenes substrates and Cu, Ni, Pd-catalysts" INDUSTRIAL CHEMISTRY LIBRARY, vol. 7, 1995, pages 240--263, XP002053319 cited in the application**
- **R. P. HOUGHTON ET AL: "Reactions of co-ordinated ligands. Part 10. Rhodium-catalysed cyclisation of 3-(2-fluorophenyl)propanols to chromans" JOURNAL OF THE CHEMICAL SOCIETY, PERKIN TRANSACTIONS 1, 1984, LETCHWORTH GB, pages 925-931, XP002053320 cited in the application**
- **T. MIGITA ET AL: "The palladium-catalyzed nuceophilic substitution of aryl halides by thiolate ions" BULLETIN OF THE CHEMICAL SOCIETY OF JAPAN, vol. 53, no. 5, May 1980, TOKYO JP, pages 1385-1389, XP002053321**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- A. ZASK ET AL: "Palladium hydrides in organic synthesis. Reduction of aryl halides by sodium methoxide catalyzed by tetrakis(triphenylphosphine)palladium" JOURNAL OF ORGANIC CHEMISTRY, vol. 43, no. 8, 1978, EASTON US, pages 1619-1620, XP002053322 cited in the application
- M. PALUCKI ET AL: "Palladium-catalyzed intermolecular carbo-oxygen bond formation: a new synthesis of aryl ethers" JOURNAL OF THE AMERICAN CHEMICAL SOCIETY, vol. 119, no. 14, 9 April 1997, DC US, pages 3395-3396, XP002053323
- M. PALUCKI ET AL: "Synthesis of oxygen heterocycles via a palladium-catalyzed C-O bond-forming reaction" JOURNAL OF THE AMERICAN CHEMICAL SOCIETY, vol. 118, no. 42, 23 October 1996, DC US, pages 10333-10334, XP002053324 cited in the application

## Description

The present invention relates to improved methods for preparing aryl ethers which are useful intermediates and end products in pharmaceutical and agricultural applications.

It has been recently reported that aryl bromides react with simple primary and secondary amines in the presence of a palladium catalyst, supporting ligands and Na(O*t*Bu) (base) to form the corresponding arylamine in good yields. See, Guram *et al. Angew. Chem.* 34(12):1348 (1995).

Despite the recent successes with palladium-catalyzed cross-coupling reactions of Ar-X with amines, comparable coupling of aryl halides with alcohols remains elusive, and this in spite of its obvious utility in organic synthesis. Aryl ethers, including oxygen heterocycles, are prominent in a large number of pharmacologically important molecules and are found in numerous secondary metabolites.

Existing methods for the conversion of Ar-X to aryl ethers often require harsh or restrictive reaction conditions and/or the presence of activating groups on the arene ring. For example, the Cu(I)-catalyzed syntheses of aryl and vinyl ethers commonly require large amounts of freshly prepared sodium alkoxides and/or large excess of the corresponding alcohol in order to achieve reasonable yields from the corresponding aryl halides and vinyl halides. See, Keegstra *et al*. *Tetrahedron* 48(17):3633 (1992).

Cramer and Coulson also reported limited success with the Ni(II)-catalyzed synthesis of diphenyl ether using sodium phenolate at reaction temperatures greater than 200 °C. See, *J. Org. Chem.* 40(16):2267 (1975). Christau and Desmurs describe the nickel-catalyzed reactions of alcohols with aryl bromides in the presence of a base. Good yields (ca. 80%) were reported only for reactions with primary alcohols with 7 mol% nickel catalyst at 125 °C. See, *Ind. Chem Libr.* 7:240 (1995). Christau and Desmurs also reported that synthesis of aryl ethers was possible only for primary and secondary alcohols. Houghton and Voyle reported the Rh(III)-catalyzed cyclization of 3-(2-fluorophenyl)propanols to chromans activated by π-bonding to the metal center; however, the reaction required very high rhodium catalyst loading (17 mol%). See, *J. Chem. Soc. Perkin Trans. I*, 925 (1984).

Ether formation has been reported as a minor side product in the palladium-catalyzed carbonylation reactions of highly activated aromatic compound such as α-substituted quinolines. Because of the highly reactive nature of the α-site, it is possible that the reaction proceeds by direct nucleophilic substitution, without promotion or catalysis by the palladium metal center. See, Cacchi *et al. Tetrahedron Lett.* 27(33):3931(1986).

Bates *et al. J. Org.Chem.* 1982,47,4374-6, relates to a high yield benzyne synthesis of diaryl ethers in the absence of a catalyst and suggests that the reaction will not work in an aromatic solvent.

Thus there remains a need for an effective method of preparing a wide range of aryl ethers under mild conditions and in high yields. There is a further need for an efficient catalytic system with high efficiencies and turnover number for the synthesis of aryl ethers. In addition, there still remains a need for an effective method for the arylation of tertiary alkoxides.

## Summary of The Invention

The present invention provides general and attractive routes to a wide range of aryl ethers. The methods provide several improvements over methods known heretofore, namely, the efficient synthesis of aryl ethers under mild conditions and in high yields. In particular, the method of the invention may be used in coupling reactions using tertiary alcohols. In other aspects of the invention, the invention provides a class of transition metal complexes useful in the catalytic reactions of the invention which were heretofore not known to be useful in the preparation of aryl ethers.

## Brief Description Of the Invention

Figure 1. Scheme illustrating possible reaction steps in the synthesis of aryl ethers according to the method of the invention.

Figure 2. Representative first-order plots for disappearance of 4 in the THF-$d_8$ at 23 (▽), 37 (Δ), 47 (O), and 55°C (x), where $[KOCH_2CMe_3]^2$0.002 M. Error bars correspond to ± 5% integration error in the corresponding $^1$H NMR spectra.

Figure 3. Second-order plot for disappearance of 4 in THF-$d_8$ at 47°C.

Figure 4. Eyring plot for the thermolysis of 4 in THF-$d_8$ over the temperature range 23 - 57°C.

Figure 5. Potassium neopentoxide concentration dependence of the rate of reductive elimination of 4 in THF-$d^8$ at 47°C.

Figure 6. Potassium neopentoxide concentration dependence of the rate of alkozide exchange with 4 in THF-$d_8$ at 47°C.

## Detailed Description Of The Invention

*Overview*.

According to a first aspect of the present invention there is provided a method of preparing an aryl ether, comprising: reacting an alcohol with an aromatic compound comprising an activated substituent, X, in an aromatic hydrocarbon solvent, in the presence of a base and a catalyst selected from the group consisting of complexes of nickel, palladium, and platinum; wherein X is a moiety whose conjugate acid, HX, has a pKa of less than 5.0.

According to a second aspect of the present invention there is provided a method of preparing an aryl ether, comprising: reacting an alkoxide salt with an aromatic compound comprising an activated substituent, X, in an aromatic hydrocarbon solvent, in the present of a catalyst selected from the group consisting of complexes of nickel, palladium, and platinum; wherein X is a moiety whose conjugate acid, HX, has a pKa of less than 5.0.

In preferred embodiments, the subject synthetic reaction can be characterized by the general reaction schemes (Scheme 1a) :

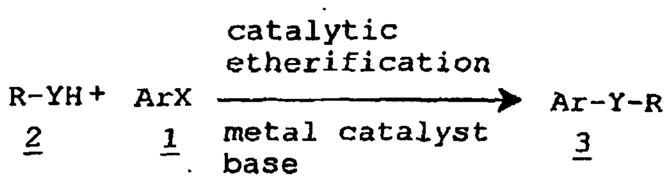


**Scheme 1a:**

wherein: Ar represents an aryl group (which may be furthered substituted beyond X): X represents a leaving group (such as a halide or a sulfonate), which can be displaced by a nucleophilic alcohol oxygen, such as in a metal-dependent etherification reaction; Y represents O; R represents, as valence and stability permit, a substituted or unsubstituted alkyl or alkenyl group, or-$(CH_2)_m$-$R_8$ wherein $R_8$ represents a substituted or unsubstituted aryl, cycloalkyl, cycloalkenyl, heterocycle or polycycle, and m is zero or an integer in the range of 1 to 8.

According to scheme 1a, an alcohol 2 (e.g., Y=O) is reacted with an aromatic compound 1 having an activated substituent, X, to form an aryl ether 3. The reaction is run in the presence of at least a catalytic amount of a transition metal catalyst which promotes the cross-coupling of the alcohol and activated aryl nucleus to form the resulting ether product, 3. The reaction, in general, proceeds in the presence of a transition metal complex (with or without a supporting ligand) and a suitable base.

The reaction may be either an intermolecular or intramolecular reaction. In the instance of the latter, it will be realized that, with reference to scheme 1a, RYH is a subsituent of Ar, and the reaction scheme can be represented by the formula:

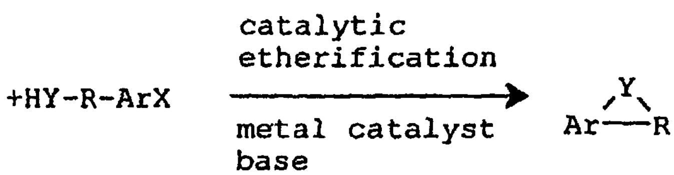


wherein R-YH represents a substituent of Ar, e.g., a hydroxy-subsitituted alkyl or alkenyl group, which disposes Y from 2 to 10 bond lengths away from the substituted position on Ar, more preferably from 2 to 5 bond lengths. Thus, upon intramolecular etherification. Y is bonded to R and the X-substituted site on Ar. Preferably, Y, R and the bridging portion of Ar form a fused ring with Ar having from 4 to 8 atoms in the ring, more preferably 5, 6 or 7 atoms. Recast slightly, the intermolecular and intramolecular reaction involving an aryl substrate can be represented in the two schemes:

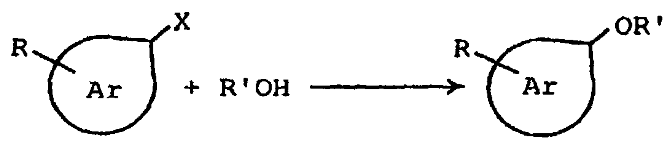

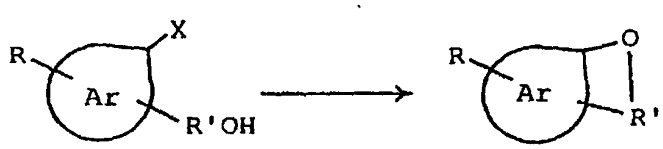

While not intending to be bound by any particular theory, the reaction most likely proceeds via oxidative-addition of the aromatic compound **2** to a zero-valent catalyst metal center, substitution of X by the alcohol **1** at the metal center, followed by reductive-elimination to generate the aryl ether **3.** The base presumably promotes formation of an oxygen-metal bond, in which the metal is the metal center of the catalyst, presumably by facilitating proton abstraction from the alcohol hydrogen.

While not being bound by any particular mode of operation, it is hypothesized that the mechanism of the preferred Pd-catalyzed synthesis of aryl ethers may proceed via a pathway similar to depicted in Figure 1. Figure 1 presents a proposed reaction pathway for the synthesis of an aryl ether via an intermolecular reaction. Any ligands that may be present on the palladium atom during this process have been omitted for clarity. With reference to the Figure, oxidative addition of the Pd(0) complex to the aryl halide affords the Pd(II) organometallic complex intermediate **A**. In the presence of a suitable base, reaction of the alcohol (or alkoxide) moiety with **A** could, after a deprotonation event to generate **B**, afford intermediate **C**, which would then undergo reductive elimination to yield the product aryl ether and regenerate the active catalyst. The reaction sequence is likely to be similar for intramolecular reactions. Alternatively, and particularly for nickel catalysts, the active transition metal species in the oxidative addition step may involve the metal in the +1 oxidation state.

In preferred embodiments of the invention, there is no need to use large excesses of either reactant - alcohol or aromatic compound. The reaction proceeds quickly and in high yield to the product aryl ether using substantially stoichiometric amount of reagents. Thus, the alcohol may be present in as little as a two-fold excess and preferably in no greater than a 20% excess relative to the aromatic compound. Alternatively, the aromatic compound may be present in as little as a two-fold excess and preferably in no greater than a 20% excess relative to the alcohol.

The reaction can proceed at mild temperatures and pressures to give high yields of the product aryl ether. Thus, yields of greater than 45%, preferably greater than 75% and even more preferably greater than 80% may be obtained by reaction at mild temperatures according to the invention. The reaction may be carried out at temperature less than 120°C, and preferably in the range of 50-120°C. In one preferred embodiment, the reaction is carried out at a temperature in the range of 80-100°C.

The reaction is carried out in an aromatic hydrocarbon solvent.

The ability to provide an ether synthesis scheme which can be carried out under mild conditions and/or with non-polar solvents has broad application, especially in the agricultural and pharmaceutical industries, as well as in the polymer industry. In this regard, the subject reaction is more amenable to use of reactants or products which include sensitive functionalities, e.g., which would otherwise be labile under harsh reaction conditions.

The subject etherification reactions can be used as part of a combinatorial synthesis scheme to yield aryl ethers. Accordingly, another aspect of the present invention relates to use of the subject method to generate variegated libraries of aryl ethers of the general formula Ar-OR, and to the libraries themselves. The libraries can be soluble or linked to insoluble supports, e.g., either through substituents of the aryl group or through R.

*__Definitions__*

For convenience, before further description of the present invention, certain terms employed in the specification, examples, and appended claims are collected here.

The term "substrate aryl group" refers to an aryl group containing an electrophilic atom which is susceptible to the subject cross-coupling reaction, e.g., the electrophilic atom bears a leaving group. In reaction scheme 1, the substrate aryl is represented by ArX, and X is the leaving group. The aryl group, Ar, is said to be substituted if, in addition to X, it is substituted at yet other positions. The substrate aryl group can be a single ring molecule, or can be a substituent of a larger molecule.

The term "reactive alcohol group" refers to an alcohol group which can attack the electrophilic atom of the substrate aryl group and displace the leaving group in the subject cross-coupling reaction. In reaction schemes 1a and 1b, the nucleophilic aryl group is represented by ROH. The reactive alcohol group can be a component of a molecule separate from the substrate aryl group, or a substituent of the same molecule (e.g., for intramolecular condensation). A "reactive thiol" and a "reactive selenol" have similar meanings.

The term "nucleophile" is recognized in the art, and as used herein means a chemical moiety having a reactive pair of electrons.

The term "electrophile" is art-recognized and refers to chemical moieties which can accept a pair of electrons from a nucleophile as defined above. Electrophilic moieties useful in the method of the present invention include halides and sulfonates.

The terms "electrophilic atom", "electrophilic center" and "reactive center" as used herein refer to the atom of the substrate aryl moiety which is attacked by, and forms a new bond to, the alcohol oxygen. In most (but not all) cases, this will also be the aryl ring atom from which the leaving group departs.

The term "electron-withdrawing group" is recognized in the art, and denotes the tendency of a substituent to attract valence electrons from neighboring atoms, i.e., the substituent is electronegative with respect to neighboring atoms. A quantification of the level of electron-withdrawing capability is given by the Hammett sigma (s) constant. This well known constant is described in many references, for instance, J. March, Advanced Organic Chemistry, McGraw Hill Book Company, New York, (1977 edition) pp. 251-259. The Hammett constant values are generally negative for electron donating groups (s[P] =-0.66 for $NH_2$) and positive for electron withdrawing groups (s[P] = 0.78 for a nitro group), s[P] indicating para substitution. Exemplary electron-withdrawing groups include nitro, ketone, aldehyde, sulfonyl, trifluoromethyl, -CN, chloride, and the like. Exemplary electron-donating groups include amino, methoxy, and the like.

The term "reaction product" means a compound which results from the reaction of the alcohol and the substrate aryl group. In general, the term "reaction product" will be used herein to refer to a stable, isolable aryl ether adduct, and not to unstable intermediates or transition states.

The term "catalytic amount" is recognized in the art and means a substoichiometric amount of reagent relative to a reactant. As used herein, a catalytic amount means from 0.0001 to 90 mole percent reagent relative to a reactant, more preferably from 0.001 to 50 mole percent, still more preferably from 0.01 to 10 mole percent, and even more preferably from 0.1 to 5 mole percent reagent to reactant.

The term "alkyl" refers to the radical of saturated aliphatic groups, including straight-chain alkyl groups, branched-chain alkyl groups, cycloalkyl (alicyclic) groups, alkyl substituted cycloalkyl groups, and cycloalkyl substituted alkyl groups. In preferred embodiments, a straight chain or branched chain alkyl has 30 or fewer carbon atoms in its backbone (e.g., $C_1$-$C_{30}$ for straight chain, $C_3$-$C_{30}$ for branched chain), and more preferably 20 or fewer. Likewise, preferred cycloalkyls have from 3-10 carbon atoms in their ring structure, and more preferably have 5, 6 or 7 carbons in the ring structure.

Moreover, the term "alkyl" (or "lower alkyl") as used throughout the specification and claims is intended to include both "unsubstituted alkyls" and "substituted alkyls", the latter of which refers to alkyl moieties having substituents replacing a hydrogen on one or more carbons of the hydrocarbon backbone. Such substituents can include, for example, a halogen, a hydroxyl, a carbonyl (such as a carboxyl, an ester, a formyl, or a ketone), a thiocarbonyl (such as a thioester, a thioacetate, or a thioformate), an alkoxyl, a phosphoryl, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. It will be understood by those skilled in the art that the moieties substituted on the hydrocarbon chain can themselves be substituted, if appropriate. For instance, the substituents of a substituted alkyl may include substituted and unsubstituted forms of amino, azido, imino, amido, phosphoryl (including phosphonate and phosphinate), sulfonyl (including sulfate, sulfonamido, sulfamoyl and sulfonate), and silyl groups, as well as ethers, alkylthios, carbonyls (including ketones, aldehydes, carboxylates, and esters), $-CF_3$, -CN and the like. Exemplary substituted alkyls are described below. Cycloalkyls can be further substituted with alkyls, alkenyls, alkoxys, alkylthios, aminoalkyls, carbonyl-substituted alkyls, $-CF_3$,-CN, and the like.

The term "aralkyl", as used herein, refers to an alkyl group substituted with an aryl group (e.g., an aromatic or heteroaromatic group).

The terms "alkenyl" and "alkynyl" refer to unsaturated aliphatic groups analogous in length and possible substitution to the alkyls described above, but that contain at least one double or triple bond respectively.

Unless the number of carbons is otherwise specified, "lower alkyl" as used herein means an alkyl group, as defined above, but having from one to ten carbons, more preferably from one to six carbon atoms in its backbone structure. Likewise, "lower alkenyl" and "lower alkynyl" have similar chain lengths. Preferred alkyl groups are lower alkyls. In preferred embodiments, a substituent designated herein as alkyl is a lower alkyl.

The term "aryl" as used herein includes 5-, 6- and 7-membered single-ring aromatic groups that may include from zero to four heteroatoms, for example, benzene, pyrrole, furan, thiophene, imidazole, oxazole, thiazole, triazole,

pyrazole, pyridine, pyrazine, pyridazine and pyrimidine, and the like. Those aryl groups having heteroatoms in the ring structure may also be referred to as "aryl heterocycles" or "heteroaromatics". The aromatic ring can be substituted at one or more ring positions with such substituents as described above, for example, halogen, azide, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, $-CF_3$, -CN, or the like. The term "aryl" also includes polycyclic ring systems having two or more cyclic rings in which two or more carbons are common to two adjoining rings (the rings are "fused rings") wherein at least one of the rings is aromatic, e.g., the other cyclic rings can be cycloalkyls, cycloalkenyls, cycloalkynyls, aryls and/or heterocyclyls.

The terms "heterocyclyl" or "heterocyclic group" refer to 3- to 10-membered ring structures, more preferably 3- to 7-membered rings, whose ring structures include one to four heteroatoms. Heterocycles can also be polycycles. Heterocyclyl groups include, for example, thiophene, thianthrene, furan, pyran, isobenzofuran, chromene, xanthene, phenoxathiin, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridinc, acridine, pyrimidine, phenanthroline, phenazine, phenarsazine, phenothiazine, furazan, phenoxazine, pyrrolidine, oxolane, thiolane. oxazole, piperidine, piperazine, morpholine, lactones, lactams such as azetidinones and pyrrolidinones, sultams, sultones, and the like. The heterocyclic ring can be substituted at one or more positions with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, $-CF_3$, -CN, or the like.

The terms "polycyclyl" or "polycyclic group" refer to two or more rings (e.g., cycloalkyls, cycloalkenyls, cycloalkynyls, aryls and/or heterocyclyls) in which two or more carbons are common to two adjoining rings, e.g., the rings are "fused rings". Rings that are joined through non-adjacent atoms are termed "bridged" rings. Each of the rings of the polycycle can be substituted with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, $-CF_3$, -CN, or the like.

The term "carbocycle", as used herein, refers to an aromatic or non-aromatic ring in which each atom of the ring is carbon.

The term "heteroatom" as used herein means an atom of any element other than carbon or hydrogen. Preferred heteroatoms are nitrogen, oxygen, sulfur and phosphorous.

As used herein, the term "nitro" means $-NO_2$; the term "halogen" designates -F,-Cl, -Br or -I; the term "sulfhydryl" means -SH; the term "hydroxyl" means -OH; and the term "sulfonyl" means $-SO_2-$.

The terms "amine" and "amino" are art recognized and refer to both unsubstituted and substituted amines, e.g., a moiety that can be represented by the general formula:

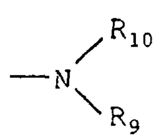
or
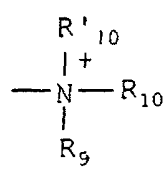

wherein $R_9$, $R_{10}$ and $R'_{10}$ each independently represent a hydrogen, an alkyl, an alkenyl, $-(CH_2)_m-R_8$, or $R_9$ and $R_{10}$ taken together with the N atom to which they are attached complete a heterocycle having from 4 to 8 atoms in the ring structure; $R_8$ represents an aryl, a cycloalkyl, a cycloalkenyl, a heterocycle or a polycycle; and m is zero or an integer in the range of 1 to 8. In preferred embodiments, only one of $R_9$ or $R_{10}$ can be a carbonyl, e.g., $R_9$, $R_{10}$ and the nitrogen together do not form an imide. In even more preferred embodiments, $R_9$ and $R_{10}$ (and optionally $R'_{10}$) each independently represent a hydrogen, an alkyl, an alkenyl, or $-(CH_2)_m-R_8$. Thus, the term "alkylamine" as used herein means an amine group, as defined above, having a substituted or unsubstituted alkyl attached thereto, i.e., at least one of $R_9$ and $R_{10}$ is an alkyl group.

The term "acylamino" is art-recognized and refers to a moiety that can be represented by the general formula:

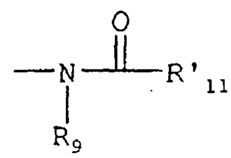

wherein R9 is as defined above, and $R'_{11}$ represents a hydrogen, an alkyl, an alkenyl or $-(CH_2)_m-R_8$, where m and $R_8$ are as defined above.

The term "amido" is art recognized as an amino-substituted carbonyl and includes a moiety that can be represented by the general formula:

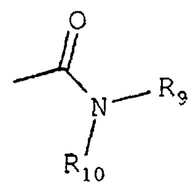

wherein $R_9$, $R_{10}$ are as defined above. Preferred embodiments of the amide will not include imides which may be unstable.

The term "alkylthio" refers to an alkyl group, as defined above, having a sulfur radical attached thereto. In preferred embodiments, the "alkylthio" moiety is represented by one of -S-alkyl, -S-alkenyl, -S-alkynyl, and $-S-(CH_2)_m-R_8$, wherein m and $R_8$ are defined above. Representative alkylthio groups include methylthio, ethyl thio, and the like.

The term "carbonyl" is art recognized and includes such moieties as can be represented by the general formula:

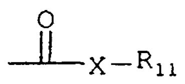 , or 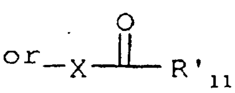

wherein X is a bond or represents an oxygen or a sulfur, and $R_{11}$ represents a hydrogen, an alkyl, an alkenyl, $-(CH_2)_m-R_8$ or a pharmaceutically acceptable salt, $R'_{11}$ represents a hydrogen, an alkyl, an alkenyl or $-(CH_2)_m-R_8$, where m and $R_8$ are as defined above. Where X is an oxygen and $R_{11}$ or $R'_{11}$ is not hydrogen, the formula represents an "ester". Where X is an oxygen, and $R_{11}$ is as defined above. the moiety is referred to herein as a carboxyl group, and particularly when $R_{11}$ is a hydrogen, the formula represents a "carboxylic acid". Where X is an oxygen, and $R'_{11}$ is hydrogen, the formula represents a "formate". In general, where the oxygen atom of the above formula is replaced by sulfur, the formula represents a "thiolcarbonyl" group. Where X is a sulfur and $R_{11}$ or $R'_{11}$ is not hydrogen, the formula represents a "thiolester." Where X is a sulfur and $R_{11}$ is hydrogen, the formula represents a "thiolcarboxylic acid." Where X is a sulfur and $R_{11}'$ is hydrogen, the formula represents a "thiolformate." On the other hand, where X is a bond, and $R_{11}$ is not hydrogen, the above formula represents a "ketone" group. Where X is a bond, and $R_{11}$ is hydrogen, the above formula represents an "aldehyde" group.

The terms "alkoxyl" or "alkoxy" as used herein refers to an alkyl group, as defined above, having an oxygen radical attached thereto. Representative alkoxyl groups include methoxy, ethoxy, propyloxy, tert-butoxy and the like. An "ether" is two hydrocarbons covalently linked by an oxygen. Accordingly, the substituent of an alkyl that renders that alkyl an ether is or resembles an alkoxyl, such as can be represented by one of -O-alkyl,-O-alkenyl, -O-alkynyl, $-O-(CH_2)_m-R_8$, where m and $R_8$ are described above.

The term "sulfonate" is art recognized and includes a moiety that can be represented by the general formula:

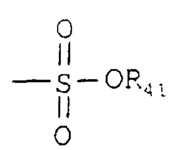

in which $R_{41}$ is an electron pair, hydrogen, alkyl, cycloalkyl, or aryl.

The term "sulfate" is art recognized and includes a moiety that can be represented by the general formula:

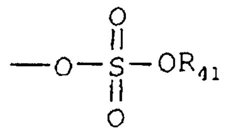

in which $R_{41}$ is as defined above.

The term "sulfonamido" is art recognized and includes a moiety that can be represented by the general formula:

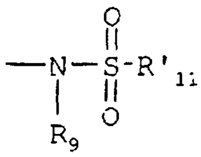

in which $R_9$ and $R'_{11}$ are as defined above.

The term "sulfamoyl" is art-recognized and includes a moiety that can be represented by the general formula:

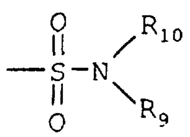

in which $R_9$ and $R_{10}$ are as defined above.

The terms "sulfoxido" or "sulfinyl", as used herein, refers to a moiety that can be represented by the general formula:

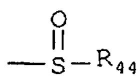

in which $R_{44}$ is selected from the group consisting of hydrogen, alkyl, alkenyl, alkynyl, cycloalkyl, heterocyclyl, aralkyl, or aryl.

A "phosphoryl" can in general be represented by the formula:

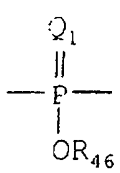

wherein $Q_1$ represented S or O, and $R_{46}$ represents hydrogen, a lower alkyl or an aryl. When used to substitute, e.g., an alkyl, the phosphoryl group of the phosphorylalkyl can be represented by the general formula:

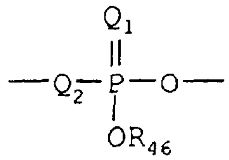, or 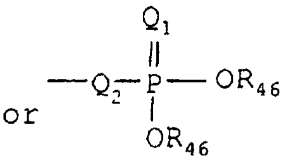

wherein $Q_1$ represented S or O, and each $R_{46}$ independently represents hydrogen, a lower alkyl or an aryl, $Q_2$ represents O, S or N. When $Q_1$ is an S, the phosphoryl moiety is a "phosphorothioate".

A "phosphoramidite" can be represented in the general formula:

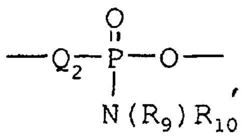, or 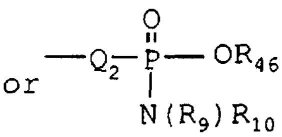

wherein $R_9$ and $R_{10}$ are as defined above, and $Q_2$ represents O, S or N.

A "phosphonamidite" can be represented in the general formula:

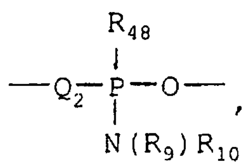, or 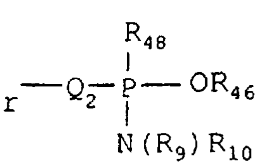

wherein $R_9$ and $R_{10}$ are as defined above, $Q_2$ represents O, S or N, and $R_{48}$ represents a lower alkyl or an aryl, $Q_2$ represents O, S or N.

A "selenoalkyl" refers to an alkyl group having a substituted seleno group attached thereto. Exemplary "selenoethers" which may be substituted on the alkyl are selected from one of -Se-alkyl, -Se-alkenyl, -Se-alkynyl, and $-Se-(CH_2)_m-R_7$, m and $R_7$ being defined above.

Analogous substitutions can be made to alkenyl and alkynyl groups to produce, for example, aminoalkenyls, aminoalkynyls, amidoalkenyls, amidoalkynyls, iminoalkenyls, iminoalkynyls, thioalkenyls, thioalkynyls, carbonyl-substituted alkenyls or alkynyls.

The phrase "protecting group" as used herein means substituents which protect the reactive functional group from undesirable chemical reactions. Examples of such protecting groups include esters of carboxylic acids, ethers of alcohols and acetals and ketals of aldehydes and ketones.

It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc.

As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described hereinabove. The permissible substituents can be one or more and the same or different for appropriate organic compounds. For purposes of this invention, the heteroatoms such as nitrogen may have hydrogen substituents and/or any permissible substituents of organic compounds described herein which satisfy the valencies of the heteroatoms. This invention is not intended to be limited in any manner by the permissible substituents of organic compounds.

A "polar solvent" means a solvent which has a dipole moment ($\varepsilon$) of 2,9 or greater, such as DMF, TMF, ethylene gylcol dimethyl ether, DMSO, acetone, acetonitrile, methanol, ethanol, isopropanol, n-propanol, t-butanol or 2-methoxyethyl ether. Preferred solvents are DMF, diglyme, and acetonitrile.

A "polar, aprotic solvent" means a polar solvent as defined above which has no available hydrogens to exchange with the compounds of this invention during reaction, for example DMF, acetonitrile, diglyme, DMSO, or THF.

An "aprotic solvent" means a non-nucleophilic solvent having a boiling point range above ambient temperature, preferably from about 25°C to about 190°C, more preferably from about 80°C to about 160°C, most preferably

from about 80°C to 150°C, at atmospheric pressure. Examples of such solvents are acetonitrile, toluene, DMF, diglyme, THF or DMSO.

For purposes of this invention, the chemical elements are identified in accordance with the Periodic Table of the Elements, CAS version, Handbook of Chemistry and Physics, 67th Ed., 1986-87, inside cover. Also for purposes of this invention, the term "hydrocarbon" is contemplated to include all permissible compounds having at least one hydrogen and one carbon atom. In a broad aspect, the permissible hydrocarbons include acyclic and cyclic, broached and unbranched, carbocyclic and heterocyclic, aromatic and nonaromatic organic compounds which can be substituted or unsubstituted.

*Exemplary Catalyzed Reactions*

As described above, the invention features a general cross-coupling reaction which comprises combining a alcohol with an aryl group (a "substrate aryl") having an electrophilic center susceptible to attack by the alcohol oxygen. In embodiments where the cross-coupling is catalyzed by a transition metal, the reaction will also include at least a catalytic amount of a transition metal catalyst and the combination is maintained under conditions appropriate for the metal catalyst to catalyze the nucleophilic addition of the reactive alcohol to the electrophilic atom of the substrate aryl.

In one embodiment, the subject method can be used to bring about formation of an intramolecular ether linkage, e.g., to form oxygen. In an exemplary embodiment, the subject method can be used to effect the intramolecular Pd-catalyzed ipso substitution of an activated aryl:

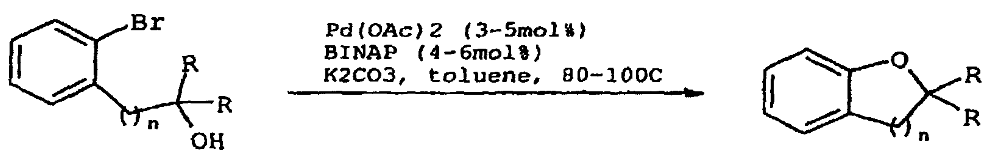


As illustrated in the appended examples, five, six and seven-membered heterocyles were obtained in good yields from the corresponding halides. In addition, a variety of functional groups were found to be compatible with the reaction conditions, including acetals, silyl ethers, and amides.

The subject method can also be used for the intermolecular formation of carbon-oxygen bonds. As an exemplary embodiment, the subject method can be used to catalyze such reactions as:

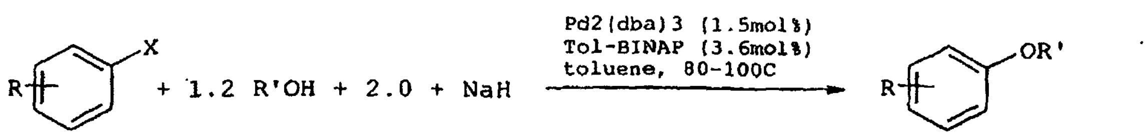


To further illustrate, the examples describe, *inter alia*, that reaction of 2-propanol, 4-bromobenzonitrile and NaH in the presence of 1.5 mol%. $Pd_2(dba)_3$ and 3 mol% (*S*)-(-)-2,2'-bis(di-*p*-tolylphosphino)-1,1'-binaphthyl (Tol-BINAP) at 50 °C afforded 4-isopropoxy-benzonitrile in 80% isolated yield.

The substrate aryl compounds include compounds derived from simple aromatic rings (single or polycylic) such as benzene, naphthalene, anthracene and phenanthrene; or heteroaromatic rings (single or polycylic), such as pyrrole, thiophene, thianthrene, furan, pyran, isobenzofuran, chromene, xanthene, phenoxathiin, pyrrole, imidazole, pyrazole, thiazole, isothiazole, isoxazole, pyridine, pyrazine, pyrimidine, pyridazine, indolizine, isoindole, indazole, purine, quinolizine, isoquinoline, quinoline, phthalazine, naphthyridine, quinoxaline, quinazoline, cinnoline, pteridine, carbazole, carboline, phenanthridine, acridine, perimidine, phenanthroline, phenazine, phenarsazine, phenothiazine, furazan, phenoxazine, pyrrolidine, oxolane, thiolane, oxazole, piperidine, piperazine, morpholine and the like. In preferred embodiment, the reactive group, X, is substituted on a five, six or seven membered ring (though it can be part of a larger polycycle).

In preferred embodiments, aryl substrate may be selected from the group consisting of phenyl and phenyl derivatives, heteroaromatic compounds, polycyclic aromatic and heteroaromatic compounds, and functionalized derivatives thereof. Suitable aromatic compounds derived from simple aromatic rings and heteroaromatic rings, include

but are not limited to, pyridinc, imidizole, quinoline, furan, pyrrole, thiophene, and the like. Suitable aromatic compounds derived from fused ring systems, include but are not limited to naphthalene, anthracene, tetralin, indole and the like.

Suitable aromatic compounds may have the formula $Z_pArX$, where X is an activated substituent. An activated substituent, X, is characterized as being a good leaving group. In general, the leaving group is a group such as a halide or sulfonate. For the purposes of the present invention, an activated substituent is that moiety whose conjugate acid, HX, has a pKa of less than 5.0. Suitable activated substituents include, by way of example only, halides such as chloride, bromide and iodide, triflate, mesylate and tosylate. In certain embodiments, the leaving group is a halide selected from iodine and bromine. Chlorine and fluorine can also be used as leaving groups, though other electronegative substitution on the aryl group may be required to activate those halogens as leaving groups in the subject metal cross-coupling reactions.

Z represents one or more optional substituents on the aromatic ring, though each occurence of Z (p>1) is independently selected. By way of example only, each incidence of substitution independently can be, as valence and stability permit, a halogen, a lower alkyl, a lower alkenyl, a lower alkynyl, a carbonyl (e.g., an ester, a carboxylate, or a formate), a thiocarbonyl (e.g., a thiolester, a thiolcarboxylate, or a thiolformate), a ketyl, an aldehyde, an amino, an acylamino, an amido, an amidino, a cyano, a nitro, an azido, a sulfonyl, a sulfoxido, a sulfate, a sulfonate, a sulfamoyl, a sulfonamido, a phosphoryl, a phosphonate, a phosphinate, $-(CH_2)_m-R_8$, $-(CH_2)_m-OH$, $-(CH_2)_m$-O-lower alkyl,$-(CH_2)_m$-O-lower alkenyl, $-(CH_2)_m-O-(CH_2)_n-R_8$, $-(CH_2)_m-SH$, $-(CH_2)_m$-S-lower alkyl, $-(CH_2)_m$-S-lower alkenyl, $-(CH_2)_m-S-(CH_2)_n-R_8$, or protecting groups of the above or a solid or polymeric support; $R_8$ represents a substituted or unsubstituted aryl, aralkyl, cycloalkyl, cycloalkenyl, or heterocycle; and n and m are independently for each occurrence zero or an integer in the range of 1 to 6. P is preferably in the range of 0 to 5. For fused rings, where the number of substitution sites on the aryl group increases, p may be adjusted appropriately.

In certain embodiments, suitable substitients Z include alkyl, aryl, acyl, heteroaryl, amino, carboxylic ester, carboxylic acid, hydrogen group, ether, thioether, amide, carboxamide, nitro, phosphonic acid, hydroxyl, sulfonic acid, halide, pseudohalide groups, and substituted derivatives thereof, and n is in the range of 0 to 5. In particular, the reaction has been found compatible with acetals, amides and silyl ethers as functional groups. For fused rings, where the number of substitution sites on the aromatic ring increases, n may be adjusted appropriately. In addition, the above mentioned moieties may be covalently linked to an alcohol moiety in intramolecular reactions.

In preferred embodiments, the resonance structure of the aryl group Ar, or at least one substituent Z, is electron-withdrawing from the substituted position of X.

A wide variety of substrate aryl groups are useful in the methods of the present invention. The choice of substrate will depend on factors such as the alcohol to be employed and the desired product, and an appropriate aryl substrate will be apparent to the skilled artisan. It will be understood that the aryl substrate preferably will not contain any interfering functionalities. It wilt further be understood that not all activated aryl substrates will react with every alcohol.

The reactive alcohol group can be a molecule separate from the substrate aryl group, or a substituent of the same molecule (e.g., for intramolecular condensation).

The alcohol is selected to provide the desired reaction product. In general, the alcohol may be any alcohol such as, but not limited to, alkyl alcohols, including primary, secondary and tertiary alcohols, and phenols. The alcohol may be functionalized. The alcohol may be selected from a wide variety of structural types, including but not limited to, acyclic, cyclic or heterocyclic compounds, fused ring compounds or phenol derivatives. The aromatic compound and the alcohol may be included as moieties of a single molecule, whereby the arylation reaction proceeds as an intramolecular reaction.

The reactive alcohol group which is used in the subject coupling reaction can be represented by general formula ROH R represents, as valence and stability permit, a subsitituted or unsubstituted alkyl or alkenyl group, or $-(CH_2)_m-R_8$, wherein $R_8$ represents a substituted or unsubstituted aryl, cycloalkyl, cycloalkenyl, heterocycle or polycycle, and m is zero or an integer in the range of 1 to 8. In other embodiments, R is linker to a solid support. Where R is substituted, it is preferably substituted with an electron withdrawing group in a manner which would substantially reduce the nucleophilicity of the hydroxyl group. For instance, R will not include any electron withdrawing groups bonds less than two bonds from the hyroxyl-substituted carbon.

In certain embodiments, the alcohol is generated *in situ,* e.g., by conversion of a precursor under the reaction conditions.

Alternatively, the corresponding alkoxide salt, e.g., NaOR, LiOR, KOR, etc., may be prepared and used in place of the alcohol. When the corresponding alkoxide is used in the reaction, an additional base may not be required.

The active form of the transition metal catalyst is not well characterized. Therefore, it is contemplated that the "transition metal catalyst" of the present invention, as that term is used herein, shall include any transition metal catalyst and/or catalyst precursor as it is introduced into the reaction vessel and which is, if necessary, converted *in situ* into the active phase, as well as the active form of the catalyst which participates in the reaction.

In preferred embodiments, the transition metal catalyst complex is provided in the reaction mixture is a catalytic

amount In certain embodiments, that amount is in the range of 0.0001 to 20 mol%, and preferably 0.05 to 5 mol%, and most preferably 1-3 mol%, with respect to the limiting reagent, which may be either the aromatic compound or the alcohol (or alkoxide) or both, depending upon which reagent is in stoichiometric excess. In the instance where the molecular formula of the catalyst complex includes more than one metal, the amount of the catalyst complex used in the reaction may be adjusted accordingly. By way of example, $Pd_2(dba)_3$ has two metal centers; and thus the molar amount of $Pd_2(dba)_3$ used in the reaction may be halved without sacrifice to catalytic activity.

Additionally, heterogeneous catalysts containing forms of these elements are also suitable catalysts for any of the transition metal catalyzed reactions of the present invention. Catalysts containing palladium and nickel arc preferred. It is expected that these catalysts will perform similarly because they are knows to undergo similar reactions, namely oxidative-addition reactions and reductive-elimination reactions, which are thought to be involved in the formation of the aryl ethers of the present invention. However, the different ligands are thought to modify the catalyst performance by, for example, modifying reactivity and preventing undesirable side reactions.

As suitable, the catalysts employed in the subject method involve the use of metals which can mediate cross-coupling of the aryl groups ArX and the alcohol as defined above. For example, suitable metals include platinum, palladium and nickel, The particular form of the metal to be used in the reaction is selected to provide, under the reaction conditions, metal centers which are coordinately unsaturated and not in their highest oxidation state. The metal core of the catalyst should be a zero valent transition metal, such as Pd or Ni with ability to undergo oxidative addition to Ar-X bond. The zerovalent state, $M^0$, may be generated in situ from $M^{+2.}$

To further illustrate, suitable transition metal catalysts include soluble complexes of platinum, palladium and nickel. Nickel and palladium are particularly preferred and palladium is most preferred. A zero-valent metal center is presumed to participate in the catalytic carbon-oxygen bond forming sequence. Thus, the metal center is desirably in the zero-valent state or is capable of being reduced to metal(0). Suitable soluble palladium complexes include, but are not limited to, tris(dibenzylideneacetone) dipalladium [$Pd_2(dba)_3$], bis(dibenzylideneacetone) palladium [$Pd(dba)_2$] and palladium acetate. Alternatively, particularly for nickel catalysts, the active species for the oxidative-addition step may be in the metal (+1) oxidative-addition state.

Catalysts containing palladium and nickel are preferred. It is expected that these catalysts will perform comparably because they are known to undergo similar reactions, namely cross-coupling reactions, which may be involved in the formation of the aryl ethers of the present invention.

The coupling can be catalyzed by a palladium catalyst which may take the form of, to illustrate, $PdCl_2$, Pd $(OAc)_2$, $(CH_3CN)_2PdCl_2$, $Pd[P(C_6H_5)_3]_4$, and polymer supported Pd(0). In other embodiments, the reaction can be catalyzed by a nickel catalyst, such as $Ni(acac)_2$, $NiCl_2[P(C_6H_5)]_2$, Raney nickel and the like, wherein "acac" represents acetylacetonate.

In some instances, it may be necessary to include additional reagents in the reaction to promote reactivity of either the transition metal catalyst or activated aryl nucleus. In particular, it may be advantageous to include a suitable base. In general, a variety of bases may be used in practice of the present invention. The base is desirably capable of extraction of a proton to promote metal-alkoxide formation. It has not been determined if deprotonation occurs prior to or after oxygen coordination. The base may optionally be sterically hindered to discourage metal coordination of the base in those circumstances where such coordination is possible, i.e., alkali metal alkoxides. Exemplary bases include such as, for example: an alkoxides such as sodium tert-butoxide, an alkali metal amide such as sodium amide, lithium diisopropylamide or an alkali metal bis(trialkyl-silyl)amides, e.g., such as lithium bis-(trimethyl-silyl)amide or sodium bis-(trimethyl- silyl) amide, a tertiary amide (e.g. triethylamine, trimethylamine, N,N-dimethylaminopyridine, 1,5-diazabicycl[4.3.0]nonene-5 (DBN), 1,5-diazabicycl [5-4.0]undecene-5 (DBU), alkali, alkaline earth carbonate, bicarbonate or hydroxide (e.g. sodium, magnesium, calcium, barium, potassium carbonate, hydroxide and bicarbonate). By way of example only, suitable bases include NaH, LiH, KH, $K_2CO_3$, $Na_2CO_3$, $Tl_2CO_3$, $Cs_2CO_3$, K(O*t*Bu), Li(O*t*Bu), Na(O*t*Bu) K(OPh), Na(OPh), triethylamine or mixtures thereof. NaH, Na(O*t*Bu) and $K_2CO_3$ have been found useful in a wide variety of aryl ether bond forming reactions. Preferred bases include $Cs_2CO_3$, DBU, NaH, KOt-Bu, $KN(SiMe_3)_2$, NaN $(SiMe_3)_2$, and $LiN(SiMe_3)_2$.

Base is used in approximately stoichiometric proportions in reaction using alcohol. The present invention has demonstrated that there is no need for large excesses of base in order to obtain good yields of aryl ether under mild reaction conditions. No more than four equivalents and preferably no more than two equivalents are needed. Further, in reactions using the corresponding alkoxide as the reagent, there may be no need for additional base.

In this way a wide range of aryl ethers, may be prepared from available alcohols. The reaction can be accomplished using a wide range of alcohols, which are either commercially available or obtainable from conventional syntheses using a variety of methods known in the art.

As is clear from the above discussion, the products which may be produced by the etherification reaction of this invention can undergo further reaction(s) to afford desired derivatives thereof. Such permissible derivatization reactions can be carried out in accordance with conventional procedures known in the art. For example, potential derivatization reactions include esterification, oxidation of alcohols to aldehydes and acids, N-alkylation of amides,

nitrile reduction, acylation of ketones by esters, acylation of amines and the like.

*III. Reaction Conditions*

The etherification reactions of the present invention may be performed under a wide range of conditions, though it will be understood that the solvents and temperature ranges recited herein are not limitative and only correspond to a preferred mode of the process of the invention.

In general, it will be desirable that reactions are run using mild conditions which will not adversely affect the reactants, the catalyst, or the product. For example, the reaction temperature influences the speed of the reaction, as well as the stability of the reactants and catalyst. The reactions will usually be run at temperatures in the range of 25°C to 300°C, more preferably in the range 25°C to 150°C.

In general, the subject reactions are carried out in a liquid reaction medium. The reactions are run in an inert solvent, preferably one in which the reaction ingredients, including the catalyst, are substantially soluble. Suitable solvents are aromatic hydrocarbon solvents such as benzene, xylene and toluene, or combinations of two or more solvents.

The invention also contemplates reaction in a biphasic mixture of solvents, in an emulsion or suspension, or reaction in a lipid vesicle or bilayer. In certain embodiments, it may be preferred to perform the catalyzed reactions in the solid phase with one of the reactants anchored to a solid support.

In certain embodiments it is preferable to perform the reactions under an inert atmosphere of a gas such as nitrogen or argon.

The reaction processes of the present invention can be conducted in continuous, semi-continuous or batch fashion and may involve a liquid recycle operation as desired. The processes of this invention are preferably conducted in batch fashion. Likewise, the manner or order of addition of the reaction ingredients, catalyst and solvent are also not generally critical and may be accomplished in any conventional fashion.

The reaction can be conducted in a single reaction zone or in a plurality of reaction zones, in series or in parallel or it may be conducted batchwise or continuously in an elongated tubular zone or series of such zones. The materials of construction employed should be inert to the starting materials during the reaction and the fabrication of the equipment should be able to withstand the reaction temperatures and pressures. Means to introduce and/or adjust the quantity of starting materials or ingredients introduced batchwise or continuously into the reaction zone during the course of the reaction can be conveniently utilized in the processes especially to maintain the desired molar ratio of the starting materials. The reaction steps may be effected by the incremental addition of one of the starting materials to the other. Also, the reaction steps can be combined by the joint addition of the starting materials to the metal catalyst. When complete conversion is not desired or not obtainable, the starting materials can be separated from the product and then recycled back into the reaction zone.

The processes may be conducted in either glass lined, stainless steel or similar type reaction equipment. The reaction zone may be fitted with one or more internal and/or external heat exchanger(s) in order to control undue temperature fluctuations, or to prevent any possible "runaway" reaction temperatures.

Furthermore, one or more of the reactants can be immobilized or incorporated into a polymer or other insoluble matrix by, for example, derivativation with one or more of substituents of the aryl group.

*IV. Combinatorial Libraries*

The subject etherification reaction readily lends itself to the creation of combinatorial libraries of aryl ethers for the screening of pharmaceutical, agrochemical or other biological or medically-related activity or material-related qualities. A combinatorial library for the purposes of the present invention is a mixture of chemically related compounds which may be screened together for a desired property. The preparation of many related compounds in a single reaction greatly reduces and simplifies the number of screening processes which need to be carried out. Screening for the appropriate biological, pharmaceutical, agrochemical or physical property is done by conventional methods.

Diversity in the library can be created at a vareity of different levels. For instance, the substrate aryl groups used in the combinatorial reactions can be diverse in terms of the core aryl moiety, e.g., a vareigation in terms of the ring structure, and/or can be varied with respect to the other substituents.

A variety of techniques are available in the art for generating combinatorial libraries of small organic molecules such as the subject arylamines. See, for example, Blondelle et al. (1995) Trends Anal. Chem. 14:83; the Affymax U. S. Patents 5,359,115 and 5.362,899: the Ellman U.S. Patent 5,288,514: the Still et al. PCT publication WO 94/08051; Chen et al. (1994) JACS 116:2661: Kerr et al. (1993) JACS 115:252; PCT publications WO92/10092, WO93/09668 and WO91/07087; and the Lerner et al. PCT publication WO93/20242). Accordingly, a variety of libraries on the order of about 100 to 1,000,000 or more diversomers of the subject aryl ethers can be synthesized and screened for particular activity or property.

In an exemplary embodiment, a library of substituted diversomers can be synthesized using the subject alcohol cross-coupling reaction adapted to the techniques described in the Still et al. PCT publication WO 94/08051, e.g., being linked to a polymer bead by a hydrolyzable or photolyzable group e.g., located at one of the positions of the aryl group or a substituent of the alcohol. According to the Still et al. technique, the library is synthesized on a set of beads, each bead including a set of tags identifying the particular diversomer on that bead. In one embodiment, which is particularly suitable for discovering enzyme inhibitors, the beads can be dispersed on the surface of a permeable membrane, and the diversomers released from the beads by lysis of the bead linker. The diversomer from each bead will diffuse across the membrane to an assay zone, where it will interact with an enzyme assay.

## Exemplification

The invention may be understood with reference to the following examples, which are presented for illustrative purposes only and which are non-limiting. Alcohols and aromatic compounds for intermolecular reactions were all commercially available. Substrates used in intramolecular reactions were prepared using standard synthetic organic methods in about 3-5 synthetic steps. Palladium catalysts were all commercially available.

Example 1-11. Examples 1-11 demonstrate the versatility of the aryl ether synthetic route of the invention. A variety of substituted aromatic compounds with attached alcohol moieties were subjected to palladium-catalyzed cross coupling to afford variously substituted heterocyclic ethers. The starting aromatic compounds and alcohols are reported in Table 1. The reactions were carried out as described in the legend.

As shown in Table 1, five, six and seven-membered heterocycles were obtained in good yields from the corresponding aryl halide. In addition, a number of functional groups were found compatible with the reaction conditions including acetals (Example 3), silyl ethers (Example 4), and amides (Example 7). Reactions performed using method A were significantly slower (24-36 h) than reactions performed using method B (1-6 h), however, the reactions using method A were somewhat cleaner. Cyclization of the aryl iodide substrate (Example 2) was extremely slow in toluene, but in 1,4-dioxane, complete conversion occurred in 24-36 h. Two equivalents of ligand relative to palladium (P:Pd = 4) and two equivalents of base relative to substrate were used to achieve reasonable yields in the cyclization reactions of Example 11 containing a secondary alcohol. Observed side products included dehalogenation of the aryl halides and in the case of substrates containing secondary alcohols, along with the oxidation of the alcohol to a ketone.

Table 1. Pd-Catalyzed Synthesis of Cyclic Aryl Ethers

| Entry | Substrate | Method[a] | Product | Yield (%)[b] |
|---|---|---|---|---|
| 1 | 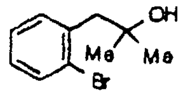 | A | 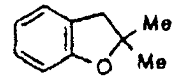 | 89 |
| 2 | 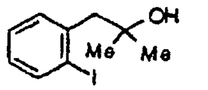 | A | 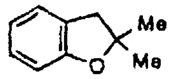 | 60 |
| 3 | 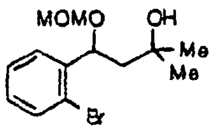 | A | 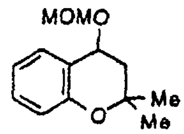 | 93 |
| 4 | 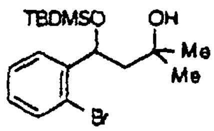 | A | 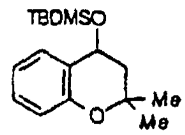 | 90 |
| 5 | 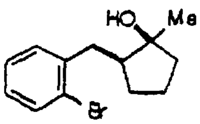 | A | 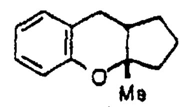 | 65 |
| 6 | 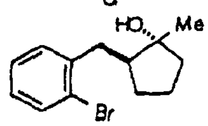 | A | 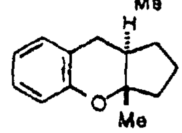 | 73 |
| 7 | 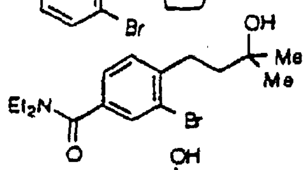 | A | 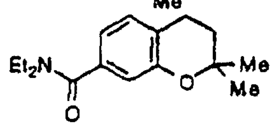 | 66 |
| 8 | 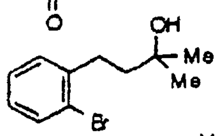 | B | 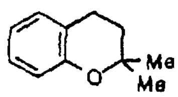 | 69 |
| 9 | 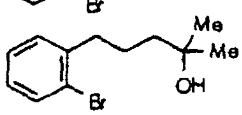 | B | 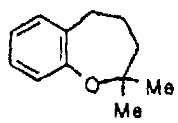 | 64 |
| 10 | 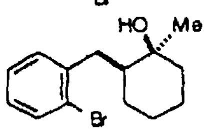 | B | 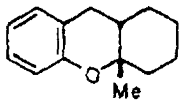 | 73 |
| 11 | 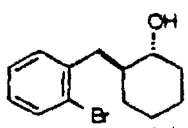 | C | 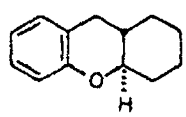 | 66 |
| 12 | 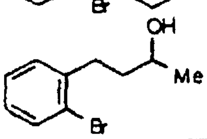 | C | 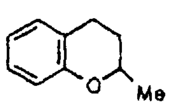 | 32 |

[a] Method A: 5 mol % $Pd(OAc)_2$, 6 mol % Tol-BINAP, 1.2 equiv of $K_2CO_3$ in toluene at 100 °C. Method B: 3 mol % $Pd(OAc)_2$, 3.6 mol % DPPF, 1.2 equiv of NaO*t*-Bu in toluene at 80 °C. Method C: 5 mol % $Pd(OAc)_2$, 10 mol % DPPF, 2.0 equiv of NaO*t*-Bu in toluene at 90 °C. [b] Yields refer to average isolated yields of two or more runs. Reaction was performed in 1,4-dioxane.

Comparative Example 1. This example demonstrates the palladium-catalyzed intermolecular synthesis of the aryl ether, 4-*t*-butoxybenzonitrile.

A Schlenk tube was charged with Na(O*t*Bu) (97 mg, 1.00 mmol), $Pd(OAc)_2$ (5.6 mg, 0.025 mmol), (*R*)-(+)-2,2'-bis(di-p-tolylphosphino)-1,1'-binaphthyl (Tol-BINAP) (20.4 mg, 0.030 mmol), 4-bromobenzonitrile (91 mg, 0.50 mmol), and toluene (3 mL). The mixture was heated at 100 °C for 30 h under an atmosphere of argon. The mixture was cooled to room temperature and diethyl ether (20 mL) and water (20 mL) were added. The organic layer was separated, washed with brine (20 mL), dried over anhydrous $MgSO_4$, and concentrated *in vacuo*. The crude product was purified by flash chromatography on silica gel (19/1 hexane/ethyl acetate) to afford 4-*t*-butoxybenzonitrile as a yellow oil (39 mg, 45% yield).

Comparative Example 2. This example demonstrates the palladium-catalyzed intermolecular synthesis of the aryl ether, 4-*t*-butylphenyl *t*-butyl ether.

An oven dried Schlenk equipped with a teflon coated stir bar was charged with Na(O*t*-Bu) (97 mg, 1.00 mmol), $Pd(OAc)_2$ (5.6 mg, 0.025 mmol), and Tol-BINAP (20.4 mg, 0.030 mmol). The Schlenk was evacuated, back-filled with argon, and charged with toluene (3 mL) and 4-*t*-butyl bromobenzene (87 µL, 0.50 mmol). The mixture was heated at 100 °C for 40 h at which time the mixture was cooled to room temperature and diethyl ether (20 mL) and water (20 mL) were added. The organic layer was separated, washed with brine (20 mL), dried over anhydrous $MgSO_4$, and concentrated *in vacuo*. The crude product was purified by flash chromatography on silica gel (99/1 hexane/ethyl acetate) to afford 4-*t*-butylphenyl *t*-butyl ether as a yellow oil (59 mg, 53% yield).

Comparative Example 3 This example demonstrates the palladium-catalyzed intermolecular synthesis of the aryl ether, 4-benzonitrile cyclopentyl ether.

A Schlenk tube was charged with NaH (80.0 mg, 60% dispersion in mineral oil, 2.00 mmol), cyclopentanol (182 µL, 2.00 mmol), and toluene (2.5 mL). The mixture was heated at 70 °C for 30 minutes under an atmosphere of argon followed by the addition of $Pd(OAc)_2$ (6.7 mg, 0.030 mmol), (*R*)-(+)-2,2'-bis(di-p-tolylphosphino)-1,1'-binaphthyl (Tol-BINAP) (27.2 mg, 0.040 mmol), 4-bromobenzonitrile (182 mg, 1.00 mmol), and toluene (2.5 mL). The mixture was heated at 100 °C for 1.5 h at which time diethyl ether (30 mL) and water (30 mL) were added at room temperature. The organic layer was separated, washed with brine (20 mL), dried over anhydrous $MgSO_4$, and concentrated *in vacuo*. The crude product was purified by flash chromatography on silica gel (19/1 hexane/ethyl acetate) to afford 4-benzonitrile cyclopentyl ether as a colorless oil (140 mg, 75% yield).

Comparative Example 4 This example demonstrates the palladium-catalyzed intermolecular synthesis of the aryl ether, 4-benzonitrile isopropyl ether.

An oven dried Schlenk tube equipped with a teflon coated stir bar was charged with NaH (60% dispersion in mineral oil, 40 mg, 1.00 mmol), placed under vacuum, and back-filled with argon. To this was added 2-propanol (46 µL, 0.60 mmol) and toluene (2 mL). The mixture was heated at 50 °C for 15 min at which time the 4-bromobenzonitrile (91 mg, 0.50 mmol), $Pd_2(dba)_3$ (6.9 mg, 0.0075 mmol), (*R*)-(+)-2,2'-bis(di-p-tolylphosphino)-1,1'-binaphthyl (Tol-BINAP) (12.2 mg, 0.018 mmol), and 1 mL of toluene were added. The mixture was heated to 50 °C while under an atmosphere of argon. After 22 h, water (50 mL) and diethyl ether (50 mL) were added and the aqueous layer separated and extracted with diethyl ether (50 mL). The organics were combined, washed with brine (50 mL) and dried over anhydrous $MgSO_4$. The crude product was purified by flash chromatography on silica gel (19:1 hexane/ethyl acetate) to afford 4-benzonitrile isopropyl ether (65 mg, 80% yield) as a white solid.

Comparative Example 5 This example demonstrates the palladium-catalyzed intermolecular synthesis of the aryl ether, 1-naphthyl cyclohexyl ether.

An oven dried Schlenk tube equipped with a teflon coated stir bar was charged with NaH (40 mg, 1.50 mmol), toluene (2 mL) and cyclohexanol (94 µL, 0.90 mmol). The mixture was heated to 70 °C for 10 min under an atmosphere of argon. To this was added 1-bromonaphthalene (104 µL, 0.75 mmol), $Pd_2(dba)_3$ (10.3 mg, 0.0113 mmol), (*R*)-(+)-2,2'-bis(di-p-tolylphosphino)-1,1'-binaphthyl (Tol-BINAP) (18.3 mg, 0.027 mmol), and 2 mL of toluene. The mixture was heated to 70 °C for 20 h at which time water (60 mL) and diethyl ether (60 mL) were added. The aqueous layer was separated and extracted with diethyl ether (60 mL). The organics were combined, washed with brine (60 mL) and dried over anhydrous $MgSO_4$. The drying agent was removed by filtration and the mother liquor concentrated *in vacuo.* The crude product was purified by flash chromatography on silica gel (50:1 hexanes:ethyl acetate) to afford 1-naphthyl

cyclohexyl ether (101 mg, 60% yield) as a colorless oil.

Comparative Example 6 This example demonstrates the palladium-catalyzed intermolecular synthesis of the aryl ether, 3-pentyl-(4-trifluoromethylphenyl) ether.

An oven dried Schlenk tube equipped with a teflon coated stir bar was charged with NaH (60% dispersion in mineral oil, 60 mg, 1.50 mmol), placed under vacuum and back-filled with argon. To this was added toluene (2 mL) and 3-pentanol (98 μL, 0.90 mmol). The mixture was heated at 70 °C for 10 min at which time 4-bromobenzotrifluoride (105 μL, 0.75 mmol), $Pd_2(dba)_3$ (10.3 mg, 0.0113 mmol), (*R*)-(+)-2,2'-bis(di-p-tolylphosphino)-1,1'-binaphthyl (Tol-BINAP) (18.3 mg, 0.027 mmol), and 1 mL of toluene were added. The mixture was heated to 70 °C for 18 h at which time diethyl ether (60 mL) and water (60 mL) were added. The aqueous layer was separated and extracted with diethyl ether (60 mL). The organics were combined, washed with brine (60 mL) and dried over $MgSO_4$. The drying agent was removed by filtration and the mother liquor concentrated *in vacuo*. The crude product was purified by flash chromatography on silica gel (19:1 hexanes:ethyl acetate) to afford 3-pentyl-(4-trifluoromethylphenyl) ether (114 mg, 54% yield) as a colorless oil.

Comparative Example 7. This example demonstrates the palladium-catalyzed intermolecular synthesis of the aryl ether, 9-anthryl cyclopentyl ether.

An oven dried Schlenk tube equipped with a teflon coated stir bar was charged with NaH (60% dispersion in mineral oil, 60 mg, 1.50 mmol), placed under vacuum and back-filled with argon. To this was added toluene (2 mL) and cyclopentanol (109 μL, 0.90 mmol). The mixture was heated at 70 °C for 15 min at which time 9-bromoanthracene (193 μL, 0.75 mmol), $Pd_2(dba)_3$ (10.3 mg, 0.0113 mmol), (*R*)-(+)-2,2'-bis(di-p-tolylphosphino)-1,1'-binaphthyl (Tol-BINAP) (18.3 mg, 0.027 mmol), and 2 mL of toluene were added. The mixture was heated at 100 °C under an atmosphere of argon. After 20 hours diethyl ether (30 mL) and brine (30 mL) were added. The organic layer was separated and dried over anhydrous $MgSO_4$. The drying agent was removed by filtration and the mother liquor concentrated *in vacuo*. The crude product was purified by flash chromatography on silica gel (99:1 hexanes:ethyl acetate) to afford 9-anthryl cyclopentyl ether (135 mg, 68% yield) as a yellow solid

Comparative Example 8 This example demonstrates the palladium-catalyzed intermolecular synthesis of the aryl ether, 4-benzonitrile benzyl ether.

An oven dried Schlenk tube equipped with a teflon coated stir bar was charged with NaH (60% dispersion in mineral oil, 60 mg, 1.50 mmol), placed under vacuum and back-filled with argon. To this was added toluene (2 mL) and benzyl alcohol (93 μL, 0.90 mmol). The mixture was heated at 70 °C for 10 min at which time 4-bromobenzonitrile (136 μL, 0.75 mmol), $Pd_2(dba)_3$ (10.3 mg, 0.0113 mmol), (*R*)-(+)-2,2'-bis(di-p-tolylphosphino)-1,1'-binaphthyl (Tol-BINAP) (18.3 mg, 0.027 mmol), and 1 mL of toluene were added. The mixture was heated at 70 °C under an atmosphere of argon. After 14 hours diethyl ether (50 mL) and water (50 mL) were added. The aqueous layer was separated and extracted with diethyl ether (50 mL). The organics were combined, washed with brine (50 mL), and dried over $MgSO_4$. The drying agent was removed by filtration and the mother liquor concentrated *in vacuo*. The crude product was purified by flash chromatography on silica gel (19:1 hexanes:ethyl acetate) to afford 4-benzonitrile benzyl ether (113 mg, 72% yield) as a white solid.

Comparative Example 9.This example demonstrates the palladium-catalyzed intermolecular synthesis of the aryl ether, 4-benzonitrile methyl ether.

An oven dried Schlenk tube equipped with a teflon coated stir bar was charged with NaH (60% dispersion in mineral oil, 60 mg, 1.50 mmol), placed under vacuum and back-filled with argon. To this was added toluene (2 mL) and methyl alcohol (87 μL, 0.90 mmol). The mixture was heated at 70 °C for 10 min at which time 4-bromobenzonitrile (136 μL, 0.75 mmol), $Pd_2(dba)_3$ (10.3 mg, 0.0113 mmol), (*R*)-(+)-2,2'-bis(di-p-tolylphosphino)-1,1'-binaphthyl (Tol-BINAP) (18.3 mg, 0.027 mmol), and 1 mL of toluene were added. The mixture was heated at 70 °C under an atmosphere of argon. After 20 hours diethyl ether (50 mL) and water (50 mL) were added. The aqueous layer was separated and extracted with diethyl ether (50 mL). The organics were combined, washed with brine (50 mL), and dried over $MgSO_4$. The drying agent was removed by filtration and the mother liquor concentrated *in vacuo*. The crude product was purified by flash chromatography on silica gel (19:1 hexanes:ethyl acetate) to afford 4-benzonitrile methyl ether (77 mg, 77% yield) as a white solid.

Comparative Example 10 : Direct Observation of C-O Reductive Elimination From Palladium(Aryl)Alkoxide Complexes to Form Aryl Ethers

Reaction of $KOCH_2CMe_3$ with [(*R*)-Tol-BINAP]Pd(*p*-$C_6H_4$CN)Br (**1**) or (dppf)Pd(*p*-$C_6H_4$CN)Br (**2**) formed the palladium(*p*-cyanophenyl)neopentoxide complexes [P-P]Pd(*p*-$C_6H_4$CN)($OCH_2CMe_3$) [P-P = Tol-BINAP (**4**), dppf(**6**)] as the exclusive products. Thermolysis of 4 in THF-$d_8$ at 55 °C formed *p*-neopentoxybenzonitrile (**5**) in 85 % yield ($t_{1/2}$ = 2.7 min). Thermolysis of **6** in THF-$d_8$ at 55 °C formed **5** in 60 % yield and pivaldehyde in 23 % yield ($t_{1/2}$ = 8.8 min). Kinetic analysis of the decomposition of **4** in the presence of excess alkoxide established the two-term rate law: Rate = $k$[**4**] + $k'$[4][$KOCH_2CMe_3$]. The alkoxide independent pathway is consistent with direct reductive elimination from **4** to generate **5** and [(*R*)-Tol-BINAP]Pd (**I**). The alkoxide dependent pathway is consistent with either alkoxide attack at palladium followed by reductive elimination from a five-coordinate palladium bis(alkoxide) complex or is consistent with attack of alkoxide at the ipso carbon atom of the palladium-bound aryl group followed by elimination of {[(*R*)-Tol-BINAP] Pd($OCH_2CMe_3$)}$^-$ **(Ia).**

Reductive elimination from a low valent group 10 metal center to form an aryl C-C bond represents the key bond forming step in a variety of synthetically relevant catalytic cross-coupling protocols.[1] As a result, the mechanisms of C-C reductive elimination from well defined Pd, Ni, and Pt complexes have been intensely investigated.[2] Similarly, C-X [X = N, S] reductive elimination presumably serves as the key bond-forming step in the corresponding palladium-catalyzed cross-coupling protocols,[3,4] and both C-N[5] and C-S[6] reductive elimination from well characterized group 10 metal complexes has been directly observed. Despite numerous examples of group 10 metal(aryl)alkoxide complexes,[7] direct thermal reductive elimination to form an aryl C-O bond has not been observed.[8,9] We have recently developed a palladium-catalyzed procedure for the formation of aryl ethers from aryl bromides and sodium alkoxides which employed mixtures of $Pd(OAc)_2$ and bulky chelating bis(phosphine) ligands such as 1,1'-bis(diphenylphosphino)ferrocene (dppf) or (*R*)-(+)-2,2'-bis(di-*p*-tolylphosphino)-1,1'-binaphthyl [(*R*)-Tol-BINAP].[10] Significantly, this system appeared to provide an opportunity to observe (aryl)C-O reductive elimination from a group 10 metal center. Here we report the generation of thermally unstable palladium(aryl)-alkoxide complexes which undergo reductive elimination to form aryl ethers.

Our approach to generate palladium(aryl)alkoxide complexes with chelating bis(phosphine) ligands involved direct displacement of the bromide ligand of a palladium (*p*-cyanophenyl)bromide complex with potassium neopentoxide. Neopentoxide was employed due to its diagnostic signals in the $^1$H NMR spectrum and because neopentanol coupled efficiently with aryl bromides under catalytic conditions.[11] The requisite palladium chelating bis(phosphine)(*p*-cyanophenyl)bromide complexes Pd[(*R*)-Tol-BINAP](*p*-$C_6H_4$CN)(Br) (**1**) and Pd(dppf)(*p*-$C_6H_4$CN)(Br) (**2**) were prepared in good yield (>75 %) from reaction of the palladium tri(*o*-tolylphosphine) dimer {Pd[P(*o*-tolyl)$_3$](*p*-$C_6H_4$CN)(μ-Br)}$_2$ (**3**) with (*R*)-Tol-BINAP or dppf, respectively. Complexes **1 - 3** were characterized by standard spectroscopic techniques and elemental analysis (Scheme 21.1).

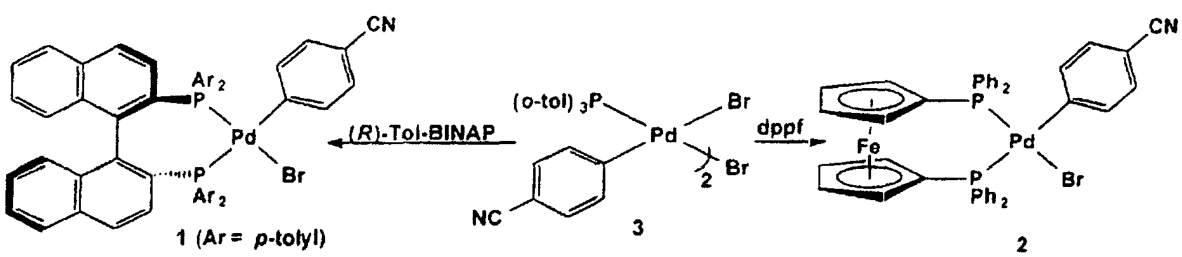


**1 (Ar = *p*-tolyl)** **3** **2**

Treatment of a pale yellow solution of **1** in THF-$d_8$ with a slight excess (~1.1 equiv.) potassium neopentoxide formed an orange solution of the palladium neopentoxide complex [(*R*)-Tol-BINAP]Pd(*p*-$C_6H_4$CN)($OCH_2CMe_3$) (**4**) in quantitative yield ($^1$H NMR; $PhSiMe_3$ internal standard). Solutions of **4** darkened within minutes at room temperature and attempts to isolate **4** from the corresponding preparative scale reaction were unsuccessful. As a result, alkoxide complex **4** was characterized by $^1$H and $^{31}$P NMR spectroscopy without isolation. The $^1$H NMR spectrum of **4** in THF-$d_8$ displayed a 1:1:1:1 ratio of *p*-tolyl peaks at δ 2.38, 2.19, 1.98, and 1.93 and a single *tert*-butyl resonance at 0.17; the ratio of these resonances established the 1:1 ratio of neopentoxide ligands to [(*R*)-Tol-BINAP]PdAr groups. A pair of doublets at 2.76 and 2.62 ($J$ = 8.8 Hz) assigned to the diastereotopic methylene protons of the alkoxide ligand confirmed binding of the alkoxide to the chiral metal fragment. The $^{31}$P NMR spectrum of **4** displayed two doublets at 25.1 and 12.1 ($J_{PP}$ = ~36 Hz), which established bidentate coordination of the phosphine ligand to the palladium alkoxide fragment.

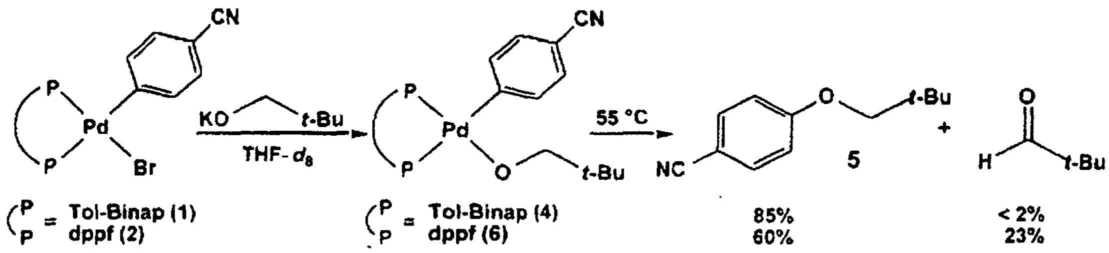


Thermolysis of a freshly prepared solution of **4** in THF-$d_8$ at 55 °C led to rapid decomposition ($t_{1/2}$ Å 2.7 min).[12] $^1$H NMR and GCMS analysis of the resulting black solution revealed the formation of *p*-neopentoxybenzonitrile (**5,** 85 %), 4,4'-dimethylbiphenyl (~10 %), and traces of benzonitrile (<5 %).[13] Products resulting from Pd/P aryl exchange[14,15] such as p-neopentoxytoluene or 4-methyl-4'-cyano-biphenyl were not observed. Thermolysis of **4** in the presence of $PPh_3$ led to no increase in yield of **5** but lowered the yield of 4,4'-dimethylbiphenyl (< 2 %). This behavior is in contrast to C-S reductive elimination from related palladium(aryl)*tert*-butylsulfide complexes which required the presence of a trapping agent to generate high yields of thioether.[5] In addition, these observations suggest that the P-C bond cleavage reaction which forms 4,4'-dimethylbiphenyl occurs subsequent to C-O reductive elimination from the reactive bis(phosphine) Pd(0) species [(*R*)-Tol-BINAP]Pd (**I**); $PPh_3$ presumably traps **I** prior to P-C oxidative addition. Thermolysis of **4** in the presence of $^3$0.04 M $KOCH_2CMe_3$ led to nearly quantitative formation of **5** ($^3$94 ± 5 %).

Addition of 1.1 eq $KOCH_2CMe_3$ to a suspension of **2** in THF-$d_8$ formed Pd(dppf)(*p*-$C_6H_4CN$)($OCH_2CMe_3$) (**6**) as the exclusive palladium species as indicated by $^1$H and $^{31}$P NMR spectroscopy; **6** was characterized by $^1$H and $^{31}$P NMR without isolation. Decomposition of neopentoxide complex **6** at 55 °C was ~ 4 times slower ($t_{1/2}$ = 8.8 min) than decomposition of **4.** $^1$H NMR and GC analysis of the resulting black solution revealed the presence of **5** (60 %), pivaldehyde (23 %), benzonitrile (30 %), and biphenyl (~10 %) (Table 21.1, Scheme 21.2). Presumably, the lower rate of reductive elimination from **6** relative to **4** leads to competitive β-hydrogen elimination in the case of **6** with formation of pivaldehyde.[16]

Table 21.1.

First-order rate constants and yields of **5** for the decomposition of **4** ([Pd] Å 1. × $10^{-2}$ M) in THF-$d_8$.

| Temp | [$KOCH_2CH_3$] | ($10^4$)$k_{obs}$ $s^{-1}$ | ArOR yield (± 5%) |
|---|---|---|---|
| 23 | 0.0017 | 1.42 ± 0.01 | 76 |
| 23 | 0.0017 | 1.43 ± 0.09 | — |
| 23 | 0,0017 | 1.45 ± 0.02 | 72[a] |
| 35 | 0.0017 | 5.2 ± 0.2 | — |
| 37 | 0.0017 | 6.3 ± 0.2 | — |
| 47 | 0.0017 | 15.7 ± 0.2 | 84 |
| 47 | 0.0017 | 14.9 ± 0.5 | 86 |
| 47 | 0.0017 | 16.1 ± 0.3 | —[b] |
| 47 | 0.043 | 11.8 ± 0.6 | 97 |
| 47 | 0.11 | 20.9 ± 0.6 | 97 |
| 47 | 0.12 | 23.0 ± 0.9 | — |
| 47 | 0.17 | 23.2 ± 0.3 | 94 |
| 47 | 0.20 | 25.5 ± 0.9 | — |
| 47 | 0.26 | 33 ± 1 | — |
| 47 | 0.28 | 33 ± 1 | — |
| 52 | 0.0017 | 30 ± 1 | 85 |
| 55 | 0.0017 | 43 ± 2 | — |
| 55 | 0.0017 | 45 ± 3 | 87[a] |
| 57 | 0.0017 | 58 ± 2 | 86 |

[a]Contained $PPh_3$ (0.15 M).
[b]Contained excess KBr

The kinetics of the reductive elimination of **4** were investigated in greater detail in an effort to probe the mechanism of palladium mediated C-O bond formation. Thermolysis of a freshly prepared solution of **4** {$[\mathbf{4}]_0$ = 17 mM} in THF-$d_8$ at 47 °C led to first-order decay of **4** over > 3 half-lives with an observed rate constant of $k_{obs} = 1.52 \pm 0.05 \times 10^{-3}$ $s^{-1}$.[12,17,18] The rate of decomposition of **4** in THF-$d_8$ was not significantly altered (<10%) by the presence of $PPh_3$ (0.15 M) or KBr (saturated) but was accelerated by addition of excess $KOCH_2CMe_3$. In order to determine the dependence of the rate on alkoxide concentration, observed rate constants for decomposition of **4** were measured as a function of $KOCH_2CMe_3$ concentration from 0.0017 to 0.30 M at 47 °C in THF-$d_8$. A plot of $k_{obs}$ versus alkoxide concentration was linear with a significant positive intercept of the ordinate which established the two-term rate law shown in eq 1, where $k = 1,50 \pm 0.07 \times 10^{-3}$ $s^{-1}$ [$\Delta G^‡ = 22.9 \pm 0.1$ kcal $mol^{-1}$] and $k' = 6.2 \pm 0.4 \times 10^{-3}$ $s^{-1}$ $M^{-1}$ [$\Delta G^‡ = 22.0 \pm 0.1$ kcal $mol^{-1}$].[17] In addition, observed rate constants for disappearance of **4** in the absence of added $KOCH_2CMe_3$[12] were measured at temperatures between 23 and 57 °C in THF-$d_8$. An Eyring plot of the data provided the activation parameters for the alkoxide independent pathway: $\Delta H‡ = 19.8 \pm 0.8$ kcal $mol^{-1}$; $\Delta S‡ = -9 \pm$ eu.[17,19]

$$\text{rate} = -\frac{-d[4]}{dt} = k[4] + k'[4][KOCH_2CMe_3] \qquad (1)$$

The neopentoxide ligand of **4** underwent facile associative exchange with free $KOCH_2CMe_3$ at 47 °C in THF-$d_8$. At low $KOCH_2CMe_3$ concentration (<2 mM), the $^1H$ NMR spectrum of **4** at 47 °C displayed a sharp *tert*-butyl resonance ($\omega_{1/2}$ < 2 Hz) with no loss of coupling between the diastereotopic benzyl protons. However, in the presence of excess $KOCH_2CMe_3$, the *tert*-butyl peak broadened considerably. Observed rate constants for alkoxide exchange were determined from excess line broadening ($\Delta\omega_{1/2} = k/\pi$)[20] as a function of alkoxide concentration from 0.0017 to 0.3 M $KOCH_2CMe_3$ at 47 °C. A plot of $k_{obs}$ versus [$KOCH_2CMe_3$] established a first-order dependence of the rate of exchange on alkoxide concentration and the second-order rate law shown in eq 2, where $k_{ex} = 1.0 \pm 0.1 \times 10^2$ $s^{-1}$ $M^{-1}$ [$\Delta G^‡$ = 15.8 ± 0.1 kcal $mol^{-1}$].[17]

$$\text{rate of alkoxide exchange} = k_{ex}\,[4][KOCH_2CMe_3] \qquad (2)$$

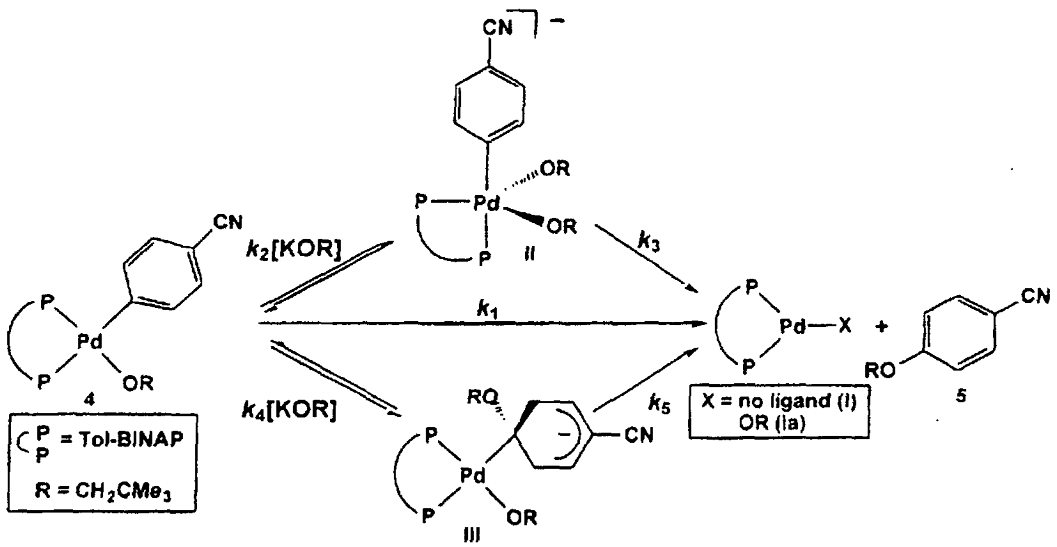

4
II
III
5

Reductive elimination from square planar $d_8$ metals to form C-C bonds has been proposed to occur from three-, four-, or five-coordinate complexes.[1] The experimental rate law for decomposition of **4** (eq 1) is consistent with C-O reductive elimination via competing alkoxide dependent and alkoxide independent pathways. The activation parameters for the alkoxide independent pathway are consistent with unimolecular reductive elimination directly from **4** to form **5** and presumably the Pd(0) species **I** (Scheme 21.3), where the empirical rate constant $k = k_1$ (Scheme 21.3).[15] We can not strictly rule out a mechanism initiated by rapid and reversible dissociation of a single phosphorus center. However, the rate of C-S reductive elimination from palladium(aryl)*tert*-butylsulfide complexes was not effected by the

rigidity of the chelating phosphine ligand, which suggested that ligand dissociation did not precede reductive elimination.[5]

The alkoxide dependent pathway could occur via rapid and reversible attack of alkoxide at palladium to generate the five-coordinate bis(alkoxide) intermediate $\{Pd[(R)\text{-Tol-BINAP}](p\text{-}C_6H_4CN)(OCH_2CMe_3)_2\}^-$ (**II**) or a related isomer.[21] Rate limiting reductive elimination from **II** could then generate **5** and presumably the three-coordinate palladium alkoxide fragment $\{[(R)\text{-Tol-BINAP}]Pd(OCH_2CMe_3)\}^-$ (**Ia**). The intermediacy of **II** is supported by the facile associative alkoxide exchange observed for **4** in the presence of $KOCH_2CMe_3$. The steady-state rate law for this pathway (eq 3) is of the same form as the second term of the experimental rate law (eq I), where $k' = k_2k_3/(k_{-2} + k_3)$. Alternatively, the alkoxide dependent pathway could occur via direct attack of alkoxide at the ipso carbon atom of the palladium-bound aryl group to form the Meisenheimer complex **III.** Collapse of **III** would then generate **5** and **Ia.** The steady-state rate law for this pathway (eq 4) is also of the same form as the second term in the experimental rate law (eq 1) where $k' = k_4k_5/(k_{-4} + k_5)$.[22]

$$\text{rate} = -\frac{d[4]}{dt} = \frac{k2k3}{k_{-2}+k_3}[4][KOCH_2CMe_3] \qquad (3)$$

$$\text{rate} = -\frac{d[4]}{dt} = \frac{k_4k_5}{k_{-4}+k_5}[4][KOCH_2CMe_3] \qquad (4)$$

In conclusion, we have presented the first examples of C-O reductive elimination from group 10 metal(aryl) alkoxide complexes to form aryl ethers. Kinetic analysis of the decomposition of **4** in the presence of excess alkoxide established a two-term rate law consistent with the presence of both an alkoxide independent and alkoxide dependent pathway for C-O reductive elimination. We continue to investigate the mechanism of this important transformation.[11b] In particular, we are probing the electronic and steric effects of both the palladium-bound aryl group and the chelating phosphine ligand on the rate and efficiency of C-O reductive elimination.

*Experimental Protocol for Comparative Example 9*

**General Methods.** All manipulations and reactions were performed under an atmosphere of nitrogen or argon in a glovebox or by standard Schlenk techniques. Preparative scale reactions were performed in flame or oven dried Schlenk tubes equipped with a stir bar, side arm joint, and a septum. NMR spectra were obtained in oven dried 5 mm thin-walled NMR tubes capped with a rubber septum on a Varian XL-300 spectrometer at 22 °C unless otherwise noted. Gas chromatography was performed on a Hewlett-Packard model 5890 gas chromatograph using a 25 m polymethylsiloxane capillary column. GC response factors were determined from mixtures of pure compounds. Elemental analyses were performed by E+R Microanalytical Laboratories (Corona, NY). Diethyl ether, hexane, and TNF-$d_8$ were distilled from solutions of sodium/benzophenone ketyl under argon or nitrogen. $Pd_2(DBA)_3$, $P(o\text{-tol})_3$, (*R*)-Tol-BINAP, dppf (Strem), 4-bromobenzonitrile, pivaldehyde, biphenyl, 4,4'-dimethylbiphenyl, and benzonitrile (Aldrich) were used as received. $KOCH_2CMe_3$, was synthesized from reaction of anhydrous neopentanol (Aldrich) and 1 equiv KH in THF.

**Kinetic Measurements.** Samples for kinetic analysis were prepared from stock solutions of the appropriate palladium aryl halide complex and were performed in oven dried 5 mm thin-walled NMR tubes capped with rubber septa. Solvent volume in the NMR tubes was calculated from the solvent height measured at 25 °C according to the relationship V (mL) = H (mm) × 0.01384 - 0.006754 and from the temperature dependence of the density of benzene.[23] Kinetic data was obtained by $^1H$ NMR spectroscopy in the heated probe of a Varian XL-300 spectrometer. Probe temperatures were measured with an ethylene glycol thermometer and were maintained at ± 0.5 °C throughout data acquisition. Syringes employed in measuring liquids for kinetic measurements were calibrated by mercury displacement and were accurate to > 95 %. Error limits for rate constants refer to the standard deviation of the corresponding least-squares-fit line.

**$\{Pd[P(o\text{-tolyl})_3](p\text{-}C_6H_4CN)(\mu\text{-}Br)\}_2$ (3).** A purple solution of $Pd_2(DBA)_3$ (1.0 g, 1.1 mmol), $P(o\text{-tol})_3$ (1.3 g. 4.3 mmol) and p-bromobenzonitrile (2.0 g, 11 mmol) in benzene (60 mL) was stirred at room temperature for 1 h. The resulting green/brown solution was filtered through Celite and benzene was evaporated under vacuum. The oily residue was dissolved in $Et_2O$ (25 mL) and allowed to stand at room temperature overnight. The resulting yellow precipitate was filtered, washed with $Et_2O$ and dried under vacuum to give **3** (0.95 g, 75 %) as a yellow powder. $^1H$ NMR ($CHCl_3$, 55 °C): δ 7.33, 7.13, 6.90, 6.73, 2.10. $^{31}P\{^1H\}$ NMR ($CDCl_3$, 55 °C): δ ~22.5 (br s). IR (THF): $\nu_{[C\equiv N]}$ 2222 $cm^{-1}$. Anal: calcd. (found) for $C_{56}H_{50}Br_2N_2P_2Pd_2$: C, 56.73 (56.57); H, 4.25 (4.51).

**$Pd[(R)\text{-Tol-BINAP}](p\text{-}C_6H_4CN)(Br)$ (1).** A solution of **3** (200 mg, 0.17 mmol), and (R)-Tol-BINAP (240 mg, 0.35 mmol) in $CH_2Cl_2$ (10 mL) was stirred at room temperature for 5 h and then evaporated under vacuum. The oily

residue was dissolved in $Et_2O$ (10 mL) and allowed to stand at room temperature for 4 h. The resulting precipitate was filtered, washed with $Et_2O$ and dried under vacuum to give **1** (308 mg, 94 %) as a yellow solid which contained traces of ether (<5 %) by $^1H$ NMR analysis. $^1H$ NMR (THF-$d_8$): δ 8.26 (dd, *J* = 8.73, 10.5), 8.04, (t, *J* = 8.2 Hz), 7.98 (t, *J* = 9.3 Hz), 7.91 (q, *J* = 7.7 Hz), 7.77 - 7.60 (m, 7 H), 7.38 (m, 4 H), 7.28 - 7.14 (m, 5 H), 6,90 (d, *J* = 8.4 Hz, 1 H). 6.77 (d, *J* = 6.8 Hz, 2 H, $C_6H_4CN$), 6.67 (d, *J* = 7.0 Hz, 2 H, $C_6H_4CN$), 2.76 (s, 3 H, *p*-tolyl), 2.56, (s, 3 H, *p*-tolyl), 2.31, (s, 3 H, *p*-tolyl), 2.29 (s, 3 H, *p*-tolyl). $^{31}P$ {$^1H$} NMR: δ 26.7 (d, *J* = 38.1 Hz), 11.4 (d, *J* = 37.9 Hz). IR (THF): $\nu_{[C\equiv N]}$ 2219 $cm^{-1}$. Anal: calcd. (found) for $C_{55}H_{44}BrNP_2Pd$: C, 68.30 (68.36); H, 4.59 (4.87).

**Pd(dppf)(*p*-$C_6H_4$CN)(Br) (2).** A solution of 3 (200 mg, 0.17 mmol), and dppf (258 mg, 0.47 mmol) in $CH_2Cl_2$ (10 mL) was stirred at room temperature for 12 h. The resulting solution was concentrated under vacuum. Addition of $Et_2O$ (10 mL) formed a precipitate which was filtered, washed with $Et_2O$ and dried under vacuum to give **2** (280 mg, 79 %) as a bright yellow solid which contained traces of ether (<5 %) by $^1H$ NMR analysis. $^1H$ NMR ($CDCl_3$): δ 8.01 (dt, *J* = 2.7, 9.7 Hz), 7.47 (m, 6 H), 7.33 (t, *J* = 11.2 Hz, 6 H), 7.12 (dt, *J* = 1.7, 15.4 Hz, 6 H), 6.76 (d, *J* = 7.54 Hz, 2 H), 4.68 (d, *J* = 1.95 Hz, 2 H, Cp), 4.51 (s, 2 H, Cp), 4.14 (d, *J* = 2.23 Hz, 2 H, Cp), 3.59 (d, J = 1.74 Hz, 2 H, Cp). $^{31}P$ {$^1H$} NMR($CDCl_3$): δ 30.0 (d, *J* = 29.2 Hz), 10.8 (d, *J* = 31.6 Hz). IR ($CH_2Cl_2$): $\nu_{[C\equiv N]}$ 2220 $cm^{-1}$. Anal: calcd. (found) for $C_{41}H_{32}BrFeNP_2Pd$: C, 58.43 (58.41); H, 3.83 (3.98).

**Pd[(*R*)-Tol-BINAP](*p*-$C_6H_4$CN)($OCH_2CMe_3$) (4).** A 0.54 M solution of $KOCH_2Me_3$ in THF-$d_8$ (25 μL, 1.35 × $10^{-2}$ mmol) was added via syringe to a colorless solution of **2** (12 mg, 1.25 × $10^{-2}$ mmol) and $PhSiMe_3$ (1.75 mg, 1.16 × $10^{-2}$ mmol) in THF-$d_8$ (0.70 mL). The tube was shaken briefly at room temperature and centrifuged to form an orange solution of **4** in quantitative (98 ± 5 %) yield by $^1H$ NMR spectroscopy versus $PhSiMe_3$ internal standard. **4** was thermally unstable and was analyzed without isolation by $^1H$ and $^{31}P$ NMR spectroscopy. $^1H$ NMR (THF-$d_8$): in addition to a small resonance corresponding to free alkoxide (δ 0.85), resonances were observed at δ 7.83 (t, *J* = 8.4 Hz), 7.78 - 7.59 (m), 7.51 - 7.25 (m), 7.12 (t, *J* = 7.4 Hz), 7.04-6.93 (m), 6.81 (d, *J* = 7.0 Hz), 6.44 (d, *J* = 7.4 Hz), 6.29 (d, *J* = 7.4 Hz) 2.76 (d, *J* = 9.0 Hz, 1 H, -$OCH_2CMe_3$), 2.62 (d, *J* = 8.8 Hz, 1 H,-$OCH_2CMe_3$), 2.38 (s, 3 H, *p*-tolyl), 2.19 (s, 3 H, *p*-tolyl), 1.98 (s, 3 H, *p*-tolyl), 1.93 (s, 3 H, *p*-tolyl), 0.17 (s, 9 H, C*Me*$_3$). $^{31}P$ {$^1H$} NMR (THF-$d_8$): δ 29.3 (d, *J* = 36.6 Hz), 14.1 (d, *J* = 36.7 Hz). IR (THF): $\nu_{[C\equiv N]}$ 2218 $cm^{-1}$.

**Pd(dppf)(*p*-$C_6H_4$CN)($OCH_2CMe_3$) (6).** A yellow suspension of **2** (11 mg, 1.3 × $10^{-2}$ mmol) in THF-$d_8$ (0.70 mL) was treated with aliquots of $KOCH_2Me_3$ in THF-$d_8$. Addition of 1.1 equiv alkoxide formed an orange solution of **6** and a small amount of free $KOCH_2Me_3$ as the exclusive products by $^1H$ NMR spectroscopy. Despite the relatively slow decomposition of **6** at room temperature ($t_{1/2}$ Å 4 h) attempts to isolate **6** from the corresponding preparative scale reaction gave only impure brown solids. $^1H$ NMR (22 °C, THF-$d_8$): δ 8.21 (m, 4 H), 7.46 (m), 7.40 (d *J* = 11.5 Hz), 7.37 (dd, *J* = 1.2, 11.6 Hz), 7.31 (t, *J* = 7.3 Hz), 7.09 (dt, *J* = 2.1, 8.0 Hz), 6.75 (dd, *J* = 2.2, 8.2 Hz, 2 H), 4.82 (q, *J* = 2.0 Hz, 2 H, Cp), 4.58 (br s, 2 H, Cp), 4.20 (t, *J* = 1.6 Hz, 2 H, Cp), 3.55 (q, *J* = 1.8 Hz, 2 H, Cp), 2.68 (s, 2 H, OC*H*$_2$CMe$_3$), 0.23 (s, 9 H, OCH$_2$C*Me*$_3$). $^{31}P$ {$^1H$} NMR (22 °C, THF-$d_8$): δ 30.9 (d, *J* = 32 Hz), 11.9 (d, *J* = 31.7 Hz). IR (THF): $\nu_{[C\equiv N]}$ 2218 $cm^{-1}$.

**Kinetics of Thermolysis of 4.** An NMR tube containing a freshly prepared solution of **4** (12 mg, 1.2 × $10^{-2}$ mmol) and $PhSiMe_3$ (1.75 mg, 1.16 × $10^{-2}$ mmol) in THF-$d_8$ (0.70 mL) {[$KOCH_2CMe_3$] Å 1.7 mM} was placed in the probe of an NMR spectrometer heated at 47 °C. The concentrations of **4**, and *p*-neopentoxybenzonitrile, were determined by integrating the *tert*-butyl resonances for **4** (δ 0.17) and *p*-neopentoxybenzonitrile (6 1.06) versus the trimethylsilyl resonance of $PhSiMe_3$ (δ 0.25) in the $^1H$ NMR spectrum. The concentration of 4,4'-dimethylbiphenyl and benzonitrile were determined by integration of the peaks for 4,4'-dimethylbiphenyl, benzonitrile, and $PhSiMe_3$ in the GC spectrum. The first-order rate constant for disappearance of **4** was determined from a plot of In [**4**] versus time (Figure 2, Table 21.1). The corresponding plot of reciprocal concentration versus time (Figure 3) deviated considerably from linearity.

First-order rate constants for disappearance of **4** in the absence of added and $KOCH_2CMe_3$ (<2 mM) were also obtained at 23, 35, 37, 52, and 57 °C (Figure 2, Table 21.1); activation parameters were obtained from a plot In [*k*/T] versus 1/T (Figure 4). First-order rate constants for disappearance of **4** were also measured as a function of [$KOCH_2CMe_3$] from 0.043 to 0.30 M at 47 °C in THF-$d_8$ (Table 21.1). Solutions of **4** with $KOCH_2CMe_3$ concentrations ranging from 0.43 to 0.12 M were obtained by a procedure analogous to that described above. Solutions of **4** with $KOCH_2CMe_3$ concentrations > 0.12 M were prepared by adding (via syringe) a THF-$d_8$ solution of **4** (17 mM) to an NMR tube containing solid $KOCH_2CMe_3$. The first-order rate constant *k* was obtained as the intercept of a plot of $k_{obs}$ versus [$KOCH_2CMe_3$] (Figure 5). The second-order rate constant *k*' was obtained from the slope of this plot.

**Kinetics of Alkoxide Exchange with 4.** An NMR tube containing a freshly prepared solution of **4** (12 mg, 1.2 × $10^{-2}$ mmol. 19 mM). $PhSiMe_3$ (1.75 mg, 1.16 × $10^{-2}$ mmol), and $KOCH_2CMe_3$ (3.4 mg, 0.0271 mmol, 0.043 mM) in THF-$d_8$ (0.63 mL) was placed in the probe of an NMR spectrometer heated at 47 °C. The excess line broadening ($\Delta\omega_{1/2}$) of the *tert*-butyl resonance of **4** was determined by measuring the peak width at half-height ($\omega_{1/2}$) for the *tert*-butyl resonance of **4** (δ 0.17) relative to $\omega_{1/2}$ for the trimethylsilyl resonance of $PhSiMe_3$ (δ 0.25) in the $^1H$ NMR spectrum [$\Delta\omega_{1/2}$ (**4**) = $\omega_{1/2}$ (**4**) - $\omega_{1/2}$ (PhTMS)]. Because the separation of the *tert*-butyl peaks for $PdOCH_2CMe_3$ and $KOCH_2CMe_3$ (Δν > 200 Hz) was much larger than the excess broadening of the *tert*-butyl resonance of **4** ($\omega_{1/2}$ = 5.5 Hz), the slow-

exchange approximation ($\Delta\omega_{1/2} = k_{obs}/\pi$) was employed to convert $\Delta\omega_{1/2}$ to $k_{obs}$.[24] Observed rate constants for alkoxide exchange were also determined at $KOCH_2CMe_3$ concentrations ranging from 0.0017 to 0.30 M. The second-order rate constant $k_{ex}$ for exchange of alkoxide with **4** was determined from the slope of a plot of $k_{obs}$ versus [$KOCH_2CMe_3$] (Figure 6, Table 21.2).

Table 21.2. First-order rate constants for alkoxide exchange 4 at 47 °C in THF-$d_8$.

| [$KOCH_2CH_3$] | $k_{obs}$ $s^{-1}$ | [$KOCH_2CH_3$] | $k_{obs}$ $s^{-1}$ |
|---|---|---|---|
| 0.0017 | 0.62 | 0.12 | 16 |
| 0.0082 | 1.6 | 0.17 | 18 |
| 0.019 | 3.0 | 0.20 | 22 |
| 0.030 | 4.2 | 0.26 | 26 |
| 0.043 | 6.1 | 0.28 | 29 |
| 0.11 | 17 | | |

**Kinetics of Thermolysis of 6.** An NMR tube containing a freshly prepared solution of **6** (11 mg, $1.2 \times 10^{-2}$ mmol, 18 mM) in THF-$d_8$ (0.70 mL) and mesitylene (1.72 mg, $1.14 \times 10^{-2}$ mmol) was placed in the probe of an NMR spectrometer preheated to 55 °C. The concentrations of **6**, and *p*-neopentoxybenzonitrile, and pivaldehyde were determined by integrating the *tert*-butyl resonances for **6** (δ 0.23), *p*-neopentoxybenzonitrile (δ 1.06), and pivaldehyde (δ 1.04) versus the methyl resonance of mesitylene (δ 2.12) in the $^1H$ NMR spectrum. The concentration of biphenyl and benzonitrile were determined by integration of the peaks for biphenyl, benzonitrile, and mesitylene in the GC spectrum. The first-order rate constant for disappearance of **6** ($k_{obs} = 1.33 \pm 0.04 \times 10^{-3}$ $s^{-1}$) was determined from a plot of ln [**6**] versus time.

**References for Comparative Example 9**

1) (a) Stille, J. K. *Angew. Chem. Int. Ed. Engl.* **1986,** *25*, 508. (b) Miyaura, N.; Suzuki, A. *Chem. Rev.* **1995**, *95*, 2457. (c) de Meijere, A.; Meyer, F. E. *Angew. Chem. Int. Ed. Engl.* **1994**, *33*, 2379.

2) (a) Collman, J. P.; Hegedus, L. S.; Norton, J. R.; Finke, R. G. *Principles and Applications of Organotransition Metal Chemistry*; University Science: Mill Valley, CA, 1987: p 322.

3) (a) Kosugi, M.; Ogata, T.: Terada. M.; Sano, H.; Migita, T. *Bull Chem. Soc. Jpn.* **1985**, *58*, 3657. (b) Dickens, M. J.; Gilday, J. P.; Mowlem T. J.; Widdowson, D. A. *Tetrahedron*, **1991,** *47*, 8621 and references therein.

4) (a) Wolfe, J. P.; Wagaw, S.; Buchwald, S. L. *J. Am. Chem. Soc. 1996*, **118**, 7215 and references therein. (b) Louie, J.; Hartwig, J. F. *Tetrahedron Lett.* **1995**, *36*, 3609.

5) Bãranano, D.; Hartwig, J. F. *J. Am. Chem. Soc.* **1995**, *117*, 2937.

6) (a) Driver, M. S.; Hartwig, J. F. *J. Am. Chem. Soc.* **1996,** *118*, 7217. (b) Villanueva, L. A.; Abboud, K. A.; Boncella, J. M. *Organometallics*, **1994,** *13,* 3921.

7) Bryndza, H. E.; Tam, W. *Chem. Rev.* **1988**, *88*, 1163.

8) Oxidation of nickel oxametallacycles has been demonstrated to form C($sp^3$)-O bonds. Koo, K.; Hillhouse, G. L.; Rheingold, A. L. *Organometallics*, **1995**, *14*, 456.

9) Reductive elimination from Pd and Ni to form an ester C(O)-O bond has been observed: Komiya. S.; Akai, Y.; Tanaka, K.; Yamamoto, T.; Yamamoto, A. *Organometallics* **1985**, *4*, 1130.

10) (a) Palucki, M.; Wolfe, J. P.; Buchwald, S. L. *J. Am. Chem. Soc.* **1996**, *118*, 10333. (b) Palucki, M.; Buchwald, S. L. submitted.

11) (a) Palucki, M.; Buchwald, S. L. unpublished results. (b) We investigated a range of alkoxides and will report the results of these studies in a full paper.

12) The solution contained a small amount (< 2 mM) of free $KOCH_2CMe_3$.

13) (a) The ultimate fate of the palladium is unclear. The $^1H$ NMR spectrum of the reaction mixture revealed a broad resonance at δ 2.1 possibly corresponding to (*R*)-Tol-BINAP palladium complexes while the $^{31}P$ NMR spectrum displayed a small resonance for free (R)-Tol-BINAP (δ-16.1) and a broad resonance at δ ~2. No resonances in the region expected for Pd[(R)-Tol-BINAP]$_2$ {δ ~25 for Pd[(R)-BINAP]$_2$}[13b] were observed. (b) Ozawa, F.; Kubo, A.; Hayashi, T. *Chem. Lett.* **1992**, 2177.

14) Morita, D. K.; Stille, J. K.; Norton, J. R. *J. Am. Chem. Soc.* **1995**, 117, 8576.

15) Braterman, P. S.; Cross, R. J.; Young, G. B. *J. Chem. Soc., Dalton Trans.* **1977**, 1892.

16) (a) Bryndza, H. E.; Calabrese, J. C.; Marsi, M.; Roe, D. C.; Tam, W.; Bercaw, J. E. *J. Am. Chem. Soc.* **1986,** *108*, 4805. (b) Hoffman, D. M.; Lappas, D.; Wierda, D. A. *J. Am. Chem. Soc.* **1993,** *115*, 10538. (c) Blum, O.; Milstein, D. *Angew. Chem., Int. Ed. Engl.* **1995,** *34*, 229.

17) See experimental protocol above.

18) The corresponding second-order plots (see Supporting Information) deviated significantly from linearity. Rate measurements employing different batches of **4** and $KOCH_2CMe_3$ provided values for $k_{obs}$ which differed by < 7.5%.

19) Under these conditions {[$KOCH_2CMe_3$]< 2 mM}, $k >> k'[KOCH_2CMe_3]$, and $k_{obs}$ Å $k$.

20) Bovey, F. A. *Nuclear Magnetic Resonance Spectroscopy,* 2nd Ed. Academic Press: San Diego, CA, 1988.

21) The proposed structure of **II** assumes the Tol-BINAP ligand will span an equatorial-axial sites (90°) rather than equatorial-equatorial sites (120°) in the trigonal bipyramid due to the preferred bite angle of ~92°; Hayashi, T.; Konishi, M.; Kobori, Y.; Kumada, M.; Higuchi, T.; Hirotsu, K. *J. Am. Chem. Soc.* **1984,** *106*, 158.

22) This rate law assumes rate limiting collapse of **III.** The rate law for rate limiting formation of **III,** followed by rapid collapse to form **5** (rate = $k_4$[**4**][$KOCH_2CMe_3$]) is also of the same form as the second term of the experimental rate law.

23) *International Critical Tables of Numerical Data, Physics, Chemistry, and Technology, Volume III*, Washburn, E. W., Ed.; McGraw-Hill: London, 1928; pp 29, 39,221.

24) Bovey, F. A. *Nuclear Magnetic Resonance Spectroscopy*, 2nd Ed. Academic Press: San Diego, CA, 1988)

Example 13: Palladium-Catalyzed Intermolecular Carbon-Oxygen Bond Formation: A New Synthesis of Aryl Ethers

The synthesis of aryl ethers by the intermolecular formation of a carbon-oxygen bond can be catalyzed by a combination of $Pd_2(dba)_3$ or $Pd(OAc)_2$ and Tol-BINAP in toluene. This process yields aryl ethers in moderate to good yields. While little or no conversion is seen in control reactions run in toluene, it was found for some electron-poor aryl

bromides that nucleophilic aromatic substitution could be carried out in DMF in the absence of metal catalyst under mild conditions.

Aryl ethers are ubiquitous structural constituents in pharmacologically important molecules, and, consequently, much research has been focused on their synthesis.[1]

Available methods for the synthesis of aryl ethers via direct nucleophilic or Cu(I)-catalyzed substitution of an aryl halide with an alcohol typically require high reaction temperatures and/or a large excess of the alcohol and are limited in substrate scope.[2,3,4] The need to employ HMPA, DMSO or DMF as solvent further diminishes the applicability of these methods, particularly for large-scale processes.

Recently we reported the first example of palladium-catalyzed aromatic carbon-oxygen bond formation; the intramolecular Pd-catalyzed ipso substitution of an aryl halide with an alcohol to afford oxygen heterocycles.[5,6] This method was used to synthesize five-, six- and seven-membered oxygen heterocycles in moderate to good yields.[7] We sought to determine whether a related catalyst system could be used for the synthesis of aryl ethers by the intermolecular coupling of alcohols and aryl bromides (eq 1).[8] Herein we report our initial results which demonstrate the viability of using palladium catalysis for the intermolecular formation of carbon-oxygen bonds in a process which takes place under mild conditions.

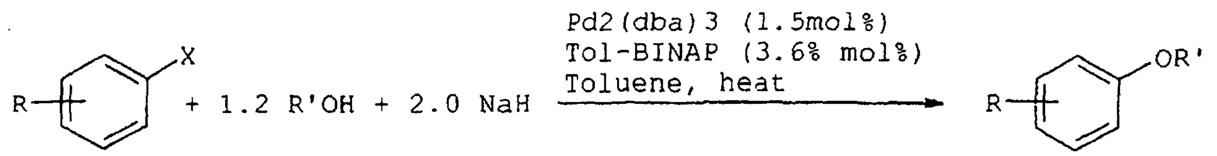


The conditions employed for the intramolecular process (vide supra) were not immediately applicable to the intermolecular version. We found, however, that reaction of 2-propanol, 4-bromobenzonitrile and NaH in the presence of 1.5 mol% $Pd_2(dba)_3$ and 3 mol% (*S*)-(-)-2,2'-bis(di-*p*-tolylphosphino)-1,1'-binaphthyl (Tol-BINAP) at 50 °C afforded 4-isopropoxy-benzonitrile in 80% isolated yield.[9] Although $Pd(OAc)_2$ was an effective catalyst precursor, use of $Pd_2(dba)_3$ afforded superior ratios of product to reduced side-product (benzonitrile) as determined by GC analyses.[10] Aryl bromides containing electron-withdrawing substituents (Table 1, entries 1-5) coupled effectively with a wide variety of alcohols including 2-propanol, 3-pentanol, (1*R*, 2*S*, 5*R*)-(-)-menthol,[11] benzyl alcohol and methanol within 24 hours using 1.5 mol% $Pd_2(dba)_3$ and 3.6 mol% Tol-BINAP at 70 °C.[12] The Pd-catalyzed coupling of methanol with 4-bromobenzonitrile is of interest since it has previously been demonstrated that methanol in combination with catalytic amounts of $Pd(PPh_3)_4$ is effective in reducing aryl halides to the dehalogenated arene products with concomitant formation of HCHO.[13] Application of this methodology using electron-rich or -neutral aryl bromides and various alcohols affords the desired coupling products in good yields only when alkoxides from tertiary alcohols or cycloalkanols are employed. For example, reaction of 4-bromo-*t*-butylbenzene with 2-propanol or cyclopentanol afforded predominantly the reduction product *t*-butylbenzene. However, reaction with NaO*t*-Bu afforded the aryl ether product in 53% isolated yield (entry 6).[14] A similar reaction of 1-bromonaphthalene with 2-propanol afforded naphthalene as the major product, while, employing cyclohexanol afforded the aryl ether product in 65% yield (entry 7). Higher ratios of aryl ether to anthracene were observed for the reaction of 9-bromoanthracene with tetrahydro-4*H*-pyran-4-ol than with cyclopentanol (entries 8 and 9).[15]

Table 22.1 Synthesis of Aryl Ethers via Pd-catalyzed and Direct Nucleophilic Substitution Reactions.

| Entry | Aryl Halide | Alcohol | Temp (°C) | Catalyzed Yield, toluene (%) [b] | Uncatalyzed Yield, DMF (%) |
|---|---|---|---|---|---|
| 1 | 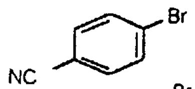 | 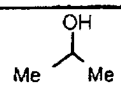 | 50 | 80 | 76 (55 °C) |
| 2 | 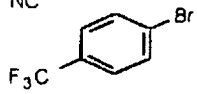 | 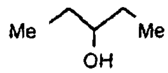 | 70 | 50 | 48 |
| 3 | 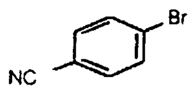 | 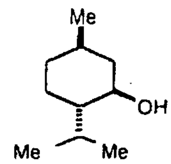 | 70 | 77 | 73 |
| 4 | 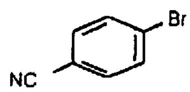 | 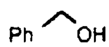 | 70 | 71 | 78 |
| 5 | 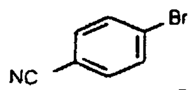 | MeOH | 70 | 81 | 65 (55 °C) |
| 6 | 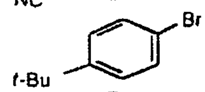 | NaO *t*-Bu | 100 | 53 | <10 |
| 7 | 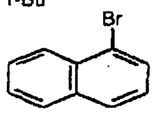 | 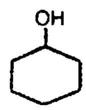 | 70 | 65 | <5 |
| 8 | 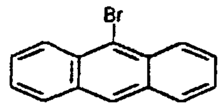 | 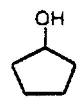 | 100 | 48 | < 10 |
| 9 | 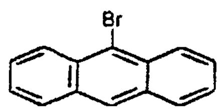 | 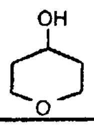 | 100 | 84 | No Rxn |

[a] For entries 1-5 and 7-9, reaction conditions: 1.5 mol% $Pd_2(dba)_3$, 3.6 mol% Tol-BINAP, 1 equiv of aryl bromide, 1.2 equiv of alcohol and 2.0 equiv of NaH. For entry 6: 5 mol% $Pd(OAc)_2$, 6 mol% Tol-BINAP, 1 equiv of aryl bromide and 2.0 equiv of NaO *t*-Bu. [b] Yields refer to the average of isolated yields for two runs.

While only preliminary studies of the mechanism of this process have been carried out, it most likely proceeds via a pathway similar to the Pd-catalyzed intramolecular C-O bond-forming reaction[5] and the related aryl amination process (Scheme 22.1a).[16] Oxidative addition of the Pd(0)Ln complex to the aryl bromide affords **A.** Substitution of the bromide with the alkoxide affords palladium(aryl)alkoxide **B.** Reductive elimination of **B** gives the aryl ether with regeneration of the active catalyst.[17] In cases which proceed in lower yields, a β-hydride elimination/reductive elimination sequence which produces the reduced arene side product is competitive with reductive elimination (Scheme 22.1b).[13c] As was observed in the aryl amination procedure, ligand effects are key to favoring the reductive-elimination process over the β-hydride elimination pathway. We feel that the use of sterically bulky and less electron-donating ligands (but probably still chelating ligands) should favor the reductive elimination process, [16b,18c]

Scheme 22.1

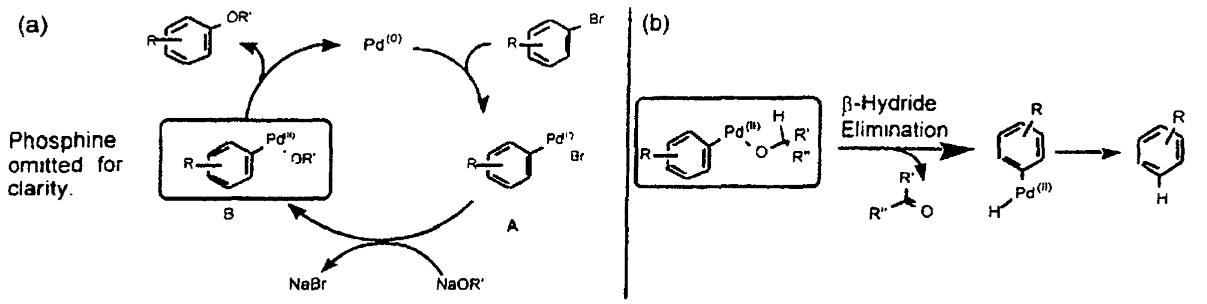


Under the conditions employed, aryl ether formation was not observed in toluene in the absence of catalyst for any of the substrates examined in Table 22.1. Since rates of nucleophilic aromatic substitution processes are enhanced in polar aprotic solvents we chose to further investigate the uncatalyzed reactions employing DMF as solvent for the substrates shown in Table 22.1.[19] In fact under these conditions, aryl bromides containing electron-withdrawing substituents could be effectively converted to aryl ethers (entries 1-5). In previous reports of the nucleophilic substitution reactions of aryl bromides with alcohols, either higher temperatures or the use of 4 or more equivalents of the alcohol were generally employed. For the substrates studied, we found that 1.2 equivalents of the alcohol was sufficient to achieve good yields of aryl ethers at 55-70 °C.[20] In contrast, in reactions of electron-neutral or -rich aryl bromides with alcohols in DMF only small amounts (<10%) of aryl ether products were observed. In addition, under these conditions, both meta and para isomers were observed in the reaction of 4-bromo-t-butylbenzene with NaOt-Bu suggesting that a benzyne pathway is operative.[21]

In order to further contrast the catalyzed and uncatalyzed processes and to extend the synthetic utility of the Pd-catalyzed transformation, the reaction of 4-bromo-2-chlorobenzonitrile was examined under both the Pd-catalyzed conditions in toluene and the uncatalyzed conditions in DMF (Table 22.2). The Pd-catalyzed reaction of4-bromo-2-chlorobenzonitrile with cyclohexanol, NaO*t*-Bu or *sec*-phenethyl alcohol in toluene afforded the aryl ether product resulting from exclusive substitution of the bromide. In the absence of a Pd-catalyst, slow substitution of chloride was observed in toluene for cyclohexanol and *sec*-phenethyl alcohol, whereas reaction with NaO*t*-Bu did not afford aryl ether products. In DMF, the uncatalyzed reaction with either cyclohexanol or *sec*-phenethyl alcohol in DMF afforded a mixture of aryl ether products while no aryl ether products were observed with NaO*t*-Bu.

**Table 22.2. Comparison of the Pd-catalyzed and Uncatalyzed Substitution of 4-Bromo-2-chlorobenzonitrile.**

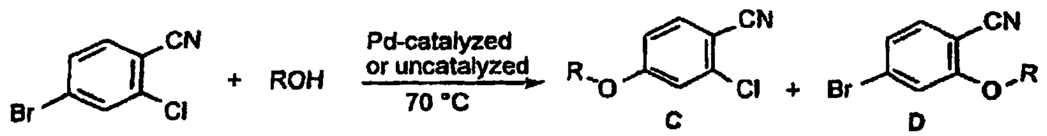


| Entry | ROH | Catalyzed Yield C (%)[a] (toluene) | Uncatalyzed Yield (%)[a] (DMF) | C/D (uncatalyzed) |
|---|---|---|---|---|
| 1[b] | 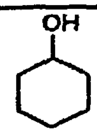 | 80 | 54 | 1:1 |
| 2[c] | 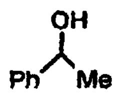 | 73 | 79 | 1:1.2 |
| 3 | NaO *t*-Bu | 82 | No rxn | -- |

[a] Yields refer to the average of isolated yield of C for two runs. [b] Isolated product from the catalyzed reaction contains 5% 4-cyclohexyloxybenzonitrile. Isolated yield for the uncatalyzed reaction refers to a mixture of C and D. [c]Isolated yield for the uncatalyzed reaction refers to the sum of the isolated yield for C and D.

The results presented above provide proof of concept that our palladium-catalyzed methodology is applicable for the formation aryl ethers by the intermolecular coupling of an aryl bromide and an alkoxide. Furthermore, our study of the uncatalyzed substitutions in both toluene (under conditions of the catalyzed process) and DMF are instructive. They indicate that the accurate comparison of the efficacies of catalyzed and uncatalyzed reactions requires the use of favorable reaction conditions for each.

*Experimental Protocol for Comparative Example* 11

**General Considerations.** All reactions were performed in oven-dried or flamedried glassware. All manipulations involving air-sensitive materials were conducted in a Vacuum Atmospheres glovebox under purified nitrogen or using standard Schlenk techniques under an atmosphere of argon. All reactions were carried out under an argon atmosphere and were stirred using a magnetic stirrer, Toluene was distilled under nitrogen from molten sodium. Sodium-*tert*-butoxide and sodium hydride (95%) were purchased from Aldrich chemical company and stored in a Vacuum Atmospheres glovebox. (*S*)-(-)-2,2'-bis(di-*p*-tolylphosphino)-1,1'-binaphthyl) (Tol-BINAP), $Pd_2(dba)_3$, $Pd(OAc)_2$, were purchased from Strem Chemical Company and used without further purification. Sodium hydride (60% dispersion in mineral oil), anhydrous methyl alcohol, cyclopentanol, 3-pentanol, (1*R*, 2*S*, 5*R*)-(-)-menthol, 9-bromoanthracene, *sec*-phenethyl alcohol, anhydrous 2-propanol, anhydrous benzyl alcohol, tetrahydro-4*H*-pyran-4-ol, menthol, 4-bromobenzonitrile, and 1-bromonaphthalene were purchased from Aldrich Chemical Company and used without further purification. 4-bromobenzotrifluoride and 4-bromo-2-chlorobenzonitrile were purchased from Lancaster Inc. and used without further purification. Cyclohexanol was purchased from Mallinckrodt Inc. and distilled over $CaH_2$ under reduced pressure. Anhydrous DMF was purchased from Aldrich Chemical Company or was distilled over $CaH_2$. Silica gel chromatographic purifications were performed by flash chromatography using E. M. Science Kieselgel 60 (230-400 mesh) silica packed in columns. Yields refer to isolated yields of compounds of greater than 95% purity as determined by capillary gas chromatography (GC) and proton Nuclear Magnetic Resonance spectroscopy ($^1H$ NMR) analysis. New compounds were also characterized by elemental analysis (E & R Analytical Laboratory, Inc). Yields reported in this section refer to a single experiment whereas those reported in Table 1 are an average of two experiments.

**General Procedures for the Pd-Catalyzed Aryl Ether Formation.** A 25 mL Schlenk flask equipped with a teflon coated stir bar was charged with NaH (1.0 mmol, 60% dispersion in mineral oil), toluene (2 mL) and the alcohol (0.50 mmol). The mixture was stirred at 70 °C for 15 min under an atmosphere of argon, cooled to room temperature, and $Pd_2(dba)_3$ (0.0075 mmol), Tol-BINAP (0.018 mmol), aryl bromide (0.50 mmol) and toluene (1 mL) were added.

The mixture was stirred at the indicated temperature until the starting material had been consumed as judged by GC analysis. At this point, the solution was cooled to room temperature and diethyl ether (50 mL) and water (50 mL) were added. The aqueous layer was separated, and extracted with diethyl ether (50 mL). The organic fractions were combined, washed with brine (50 mL), dried over anhydrous $MgSO_4$, filtered, and concentrated *in vacuo*. The crude product was purified by flash chromatography on silica gel.

**4-isopropoxybenzonitrile** (Table 1, entry 1). The general procedure was followed on a 0.50 mmol scale to afford 65 mg (80% yield) of a colorless oil. IR (neat, $cm^{-1}$) $\nu_{max}$: 2981, 2225, 1605, 1506, 1299, 1259; $^1H$ NMR ($CDCl_3$) $\delta$ 7.55 (d, *J* = 8.7 Hz, 2H), 6.91 (d, *J* = 8.8 Hz, 2H), 4.61 (septet, *J* = 6.0, 1H), 1.34 (d, *J* = 6.0 Hz, 6H); $^{13}C$ NMR ($CDCl_3$) 161.4, 133.9, 126.3, 116.0, 70.4, 21.7; Anal Calcd. for $C_{10}H_{11}NO$: C, 74.51; H, 6.88. Found: C, 74.35; H, 7.08.

**4-trifluoromethylphenyl 3-pentyl ether** (Table 1, entry 2). The general procedure was followed on a 0.75 mmol scale to afford 114 mg (54% yield) of a colorless oil. IR (neat, $cm^{-1}$) $\nu_{max}$: 2970, 1615, 1518, 1329, 1257, 1161, 1116, 1068; $^1H$ NMR ($CDCl_3$) $\delta$ 7.53 (d, *J* = 8.7 Hz, 2H), 6.96 (d, *J* = 8.7 Hz, 2H), 4.19 (quintet, *J* = 5.8 Hz, 1H), 1.65-1.77 (m, 4H) 0.97 (t, *J* = 7.4 Hz, 6H); $^{13}C$ NMR($CDCl_3$) $\delta$ 161.4, 126.9, 126.8, 126.7, 115.6, 80.5, 26.0, 9.5; Anal Calcd. for $C_{12}H_{15}F_3O$: C, 62.31; H, 6.11. Found: C, 62.04; H, 6.30.

**4-((1*R*, 2*S*, 5*R*)-menthyloxy)benzonitrile** (Table 1, entry 3). The general procedure was followed on a 0.5 mmol scale to afford 96 mg (74% yield) as a white solid, mp 73 °C; IR (neat, $cm^{-1}$) $\nu_{max}$: 2959, 2220, 1602, 1504, 1252, 988; $^1H$ NMR ($CDCl_3$) $\delta$ 7.55 (d, *J* = 8.5 Hz, 2H), 9.92 (d, *J* = 8.5 Hz, 2H), 4.10 (dt, 1H, *J* = 3.8, 10.4 Hz), 2.08-2.15 (m, 2H), 1.68-1.79 (m, 2H), 1.40-1.60 (m, 2H), 0.90-1.97 (m, 9H), 0.74 (d, J = 7.0 Hz, 3H); $^{13}C$ NMR ($CDCl_3$) 161.8, 134.0, 119.4, 115.8, 103.2, 77.8, 47.7, 39.8, 34.3, 31.3, 26.1, 23.2, 22.0, 20.6, 16.5; Anal Calcd. for $C_{17}H_{24}NO$: C, 70.33; H, 9.01. Found: C, 79.50; H, 8.91.

**4-Benzyloxybenzonitrile[22]** (Table 1, entry 4). The general procedure was followed on a 0.75 mmol scale to afford 113 mg (72% yield) of a white solid. mp 89-90 °C (lit mp 91-93 °C); IR (KBR, $cm^{-1}$) $\nu_{max}$: 2220, 1606, 1508, 1462, 1263, 1170, 1027, 837; $^1H$ NMR ($CDCl_3$) $\delta$ 7.57 (dd, *J* = 6.8, 1.9 Hz, 2H), 7.37-7.42 (m, 5H), 7.02 (dd, *J* = 6.8, 1.9 Hz, 2H), 5.11 (s, 2H); $^{13}C$ NMR ($CDCl_3$) $\delta$ 161.8, 135.6, 133.9, 128.6, 128.3, 127.3, 119.0, 115.5, 104.1, 70.2; Anal Calcd. for $C_{14}H_{11}NO$: C, 80.35; H, 5.30. Found: C, 80.60; H, 5.27.

**4-methoxybenzonitrile** (Table 1, entry 5). The general procedure was followed on a 0.75 mmol scale to afford 77 mg (77% yield) of a white solid, mp 56.0-56.8 °C (lit[23] mp 57-59 °C); $^1H$ NMR ($CDCl_3$) $\delta$ 7.57 (d, *J* = 8.9 Hz, 2H), 6.93 (d, *J* = 8.8 Hz, 2H), 3.84 (s, 3H);$^{13}C$ NMR ($CDCl_3$) $\delta$ 162.7, 133.8, 119.0, 114.6, 103.8, 55.4.

**4-*t*-Butylphenyl *t*-butyl ether** (Table 1, entry 6). A 25 mL Schlenk flask with a teflon coated stir bar was charged with NaO*t*-Bu (97 mg, 1.00 mmol), $Pd(OAc)_2$ (5.6 mg, 0.025 mmol) and Tol-BINAP (20.4 mg, 0.030 mmol). The flask was evacuated, back-filled with argon, and charged with toluene (3 mL) and 4-*t*-butyl bromobenzene (87 µL, 0.50 mmol). The mixture was heated at 100 °C for 40 h, cooled to room temperature and then diethyl ether (20 mL) and water (20 mL) were added. The organic layer was separated, washed with brine (20 mL), dried over anhydrous $MgSO_4$, and concentrated *in vacuo*. The crude product was purified by flash chromatography on silica gel (99/1 hexane/ethyl acetate) to afford 4-*t*-butylphenyl *t*-butyl ether as a colorless oil (59 mg, 53% yield). IR (neat, $cm^{-1}$) $\nu_{max}$: 2964, 1605, 1506, 1364, 1245, 1169; $^1H$ NMR ($CDCl_3$) $\delta$ 7.25 (d, *J* = 8.6 Hz, 2H), 6.89 (d, *J* = 9.0 Hz, 2H), 1.33 (s, 9H), 1.30 (s, 9H); $^{13}C$ NMR ($CDCl_3$) $\delta$ 153.0, 145.9, 77.9, 34.2, 31.5, 28.9; Anal Calcd. for $C_{14}H_{22}O$: C, 81.50; H, 10.75. Found: C, 81.59; H, 10.53.

**1-Naphthyl cyclohexyl ether** (Table 1, entry 7). A 25 mL Schlenk flask with a teflon coated stir bar was charged with NaH (40 mg, 1.50 mmol), toluene (2 mL), and cyclohexanol (94 µL, 0.90 mmol). The mixture was heated to 70 °C for 10 min under an atmosphere of argon and then cooled to room temperature. To this was added 1-bromonaphthalene (104 µL, 0.75 mmol), $Pd_2(dba)_3$ (10.3 mg, 0.0125 mmol), (*R*)-(+)-2,2'-(di-p-tolylphosphino)-1,1'-binaphthyl (Tol-BINAP) (18.3 mg, 0.027 mmol), and 2 mL of toluene. The mixture was heated to 70 °C for 20 h at which time water (60 mL) and diethyl ether (60 mL) were added. The aqueous layer separated and extracted with diethyl ether (60 mL). The organic fractions were combined, washed with brine (60 mL), and dried over anhydrous $Mg_2SO_4$. The drying agent was removed by filtration and the mother liquor concentrated *in vacuo*. The crude product was purified by flash chromatography on silica gel (50:1 hexanes:ethyl acetate) to afford 1-naphthyl cyclohexyl ether (101 mg, 60 % yield) as a colorless oil. IR (neat, $cm^{-1}$) $\nu_{max}$: 3051, 2934, 2857, 1579, 1401, 1268, 1236, 1094; $^1H$ NMR ($CDCl_3$) $\delta$ 8.25-8.29 (m, 1H), 7.69-7.36 (m, 1H), 7.28-7.43 (m, 3H), 6.77 (dd, *J* = 7.1, 1.3 Hz, 1H), 4.41 (quintet, *J* = 4.4 Hz, 1H), 1.93-1.97 (m, 2H), 1.32-1.80 (m 8H); $^{13}C$ NMR ($CDCl_3$) $\delta$ 153.5, 134.8, 127.4, 126.7, 126.2, 125.8, 124.9, 122.4, 119.9, 75.3, 31.6, 25.8, 23.6; Anal Calcd. for $C_{16}H_{18}O$: C, 84.91; H, 8.02. Found: C, 85.04; H, 7.88.

**9-Anthryl cyclopentyl ether** (Table 1, entry 8). The general procedure was followed on a 0.5 mmol scale with a reaction temperature of 100 °C to afford 58 mg (49% yield) of a white solid. mp 60-62 °C; IR (neat, $cm^{-1}$) $\nu_{max}$: 2960, 1337, 1084; $^1H$ NMR ($CDCl_3$) $\delta$ 8.29-8.32 (m, 2H), 8.19 (s, 1H), 7.95-7.99 (m, 2H), 7.41-7.48 (m, 4H), 4.90-4.96 (m, 1H), 2.00-2.20 (m, 4H), 1.65-1.85 (m, 4H); $^{13}C$ NMR ($CDCl_3$) $\delta$ 150.9, 132.4. 130.9, 128.3, 125.3, 124.7, 123.1, 121.7, 88.3, 33.1, 23.1; Anal Calcd. for $C_{19}H_{18}O$: C, 86.99; H, 6.92. Found: C, 87.2; H, 6.92

**9-Anthryl 4-tetrahydro-4*H*-pyran ether** (Table 1, entry 9). The general procedure was followed on a 0.50 mmol scale with a reaction temperature of 100 °C to afford 125 mg (90% yield) of a yellow solid, mp 99.9-100.8 °C;

IR (KBr, $cm^{-1}$) $\nu_{max}$: 3048, 2951, 2839, 1409, 1343, 1168, 1092, 1003, 737; $^1H$ NMR ($CDCl_3$) δ 8.27-8.30 (m, 2H), 8.18 (s, 1H), 7.92-7.97 (m, 2H), 7.39-7.48 (m, 4H), 4.38-4.46 (m, 1H), 3.98-4.06 (m, 2H), 3.27-3.37 (m, 2H), 1.95-2.17 (m, 4H); $^{13}C$ NMR ($CDCl_3$) δ 149.4, 132.3, 128.3, 125.4, 125.3, 125.0, 122.8, 122.1, 106.5, 80.5, 66.2, 33.6; Anal Calcd. for $C_{19}H_{18}O_2$: C, 81.99; H, 6.52. Found: C, 81.75; H, 6.66.

**4-Cyclohexyloxy-2-chlorobenzonitrile** (Table 2, entry 1). The general procedure was followed on a 0.50 mmol scale to afford 96 mg (81% yield) of a white solid, mp 30-32 °C. The isolated product contained a 5% impurity as determined by GC analysis which was identified by GC/MS as 4-cyclohexyloxybenzonitrile (GC/MS, m/z = 201) and NMR analysis. IR (neat, $cm^{-1}$) $\nu_{max}$: 2935, 2228, 1599, 1491, 1044; $^1H$ NMR ($CDCl_3$) δ 7.52-7.57 (m, 1H), 6.98 (d, *J* = 2.4 Hz, 1H), 6.80-6.85 (m, 1H), 4.31 (quintet, *J* = 3.8 Hz, 1H), 1.90-2.01 (m, 2H), 1.74-1.89 (m, 2H), 1.50-1.62 (m, 3H), 1.30-1.43 (m, 3H); $^{13}C$ NMR ($CDCl_3$) δ 161.8, 138.1, 134.9, 116.9, 116.4, 114.6, 104.3, 76.3, 31.3, 25.3, 23.4; GC/MS (m/z) 235, 237.

**2-Chloro-4-*sec*-phenethyloxybenzonitrile** (Table 2, entry 2). The general procedure was followed on a 0.50 mmol scale to afford 107 mg (83% yield) of a colorless oil. IR (neat, $cm^{-1}$) $\nu_{max}$: 3032, 2982, 2932, 2228, 1598, 1489, 1454, 1297, 1277, 1239, 1067; $^1H$ NMR ($CDCl_3$) δ 7.24-7.46 (m, 6H), 6.97 (d, *J* = 2.4 Hz, 1H), 5.33 (q, *J* = 6.4 Hz, 1H), 1.65 (d, *J* = 6.4 Hz, 3H); $^{13}C$ NMR ($CDCl_3$) δ 161.8, 141.2, 137.9, 134.8, 128.9, 128.1, 125.3, 117.5, 116.2, 114.7, 104.8, 77.5, 24.1; Anal Calcd. for $C_{15}H_{12}ClNO$: C, 70.02; H, 4.70. Found: C, 70.14; H, 4.68.

**4-*t*-Butyloxy-2-chlorobenzonitrile** (Table 2, entry 3). The general procedure was followed on a 0.50 mmol scale to afford 87 mg (83% yield) of a colorless oil. (Table 2, entry 3) IR (neat, $cm^{-1}$) $\nu_{max}$: 2980, 2228, 1595, 1484, 1238, 1160, 1041; $^1H$ NMR ($CDCl_3$) δ 7.54-7.56 (m, 1H), 7.09-7.10 (m, 1H), 6.93-6.97 (m 1H), 1.44 (s, 9H); $^{13}C$ NMR ($CDCl_3$) δ 160.6, 137.4, 134.4, 123.1, 120.5, 116.1, 106.4, 81.0, 28.7; Anal Calcd. for $C_{11}H_{12}ClNO$: C, 63.01; H, 5.77. Found: C, 62.09; H, 5.56.

**General Procedure for the uncatalyzed coupling reaction in DMF.** A 25 mL resealable Schlenk flask was charged with NaH (0.60 mmol, 60% dispersion in mineral oil), anhydrous DMF (2 mL), alcohol (0.60 mmol), and aryl halide (0.50 mmol) under an argon atmosphere. The flask was sealed and heated to the temperature indicated until the starting material had been consumed as judged by GC analysis. At this point, the solution was cooled to room temperature and diethyl ether (50 mL) and water (50 mL) were added. The aqueous layer was separated, and extracted with diethyl ether (50 mL). The organic layers were combined, washed with brine (50 mL), dried over anhydrous $MgSO_4$, filtered and concentrated *in vacuo.* The crude product was purified by flash chromatography on silica gel.

**4-Bromo-2-*sec*-phenethyloxybenzonitrile** (Table 2, entry 2). The general procedure for the uncatalyzed coupling reaction in DMF was followed on a 0.50 mmol scale to afford 61 mg (40% yield) of the title compound as a white solid and 2-chloro-4-*sec*-phenethyloxybenzonitrile (44 mg, 34% yield) as a colorless oil. mp 101-102 °C; IR (KBr, $cm^{-1}$) $\nu_{max}$: 2226, 1590, 1482, 1408, 1253, 1063, 943; $^1H$ NMR ($CDCl_3$) δ 7.29-7.39 (m, 6H), 7.07 (dd, *J* = 8.3, 1.6 Hz, 1H), 6.99 (d, 1.6 Hz, 1H), 5.39 (q, *J* = 6.4 Hz, 1H), 1.71 (d, *J*= 6.4 Hz, 1H); $^{13}C$ NMR ($CDCl_3$) δ 159.4, 140.4, 133.7, 128.3, 127.8, 127.6, 124.9, 123.7, 117.4, 115.2, 76.0, 23.6; Anal Calcd. for $C_{15}H_{12}BrNO$: C, 59.62; H, 4.00. Found: C, 59.41; H, 3.84.

**References for Comparative Example 10**

(1) For a review of alkenyl and aryl C-O bond forming reactions, see: (a) Chiuy, C. K.-F. In *Comprehensive Organic Functional Group Transformations*; Katritzky, A. R.; Meth-Cohn, O.; Rees, C. W., Ed; Pergamon Press: New York, 1995; Vol. 2, Ch. 2.13. (b) Paradisi, C. In *Comprehensive Organic Synthesis*; Trost, B. M.; Fleming, I.; Semmelhack, M. F., Ed; Pergamon Press: New York, 1991; Vol 4, Ch 2.1.

(2) (a) Lu, T.; Hyunsook, K. S.; Zhang. H.; Bott, S.; Atwood, J. L.; Echegoyen, L.; Gokel, G. W. *J. Org. Chem.* **1990,** *55*, 2269. (b) Pluta, K. *J. Heterocyclic Chem.* **1984,** *31*, 557 (c) Testaferri, L.; Tiecco, M.; Tingoli, M.; Bartoli, C.; Massoli, A. *Tetrahedron* **1985,** *41*, 1373. (d) Testaferri, L.; Tiecco, M.; Tingoli, D.; Chianelli, D.; Montanucci, M. *Tetrahedron* **1983,** *39*, 193. (e) Shaw, J. E.; Kunerth, D. C.; Swanson, S. B. *J. Org. Chem.* **1976,** *41*, 732. (f) Bradshaw, J. S.; Hales, R. H. *J. Org. Chem.* **1971,** *36*, 318

(3) (a) Lee, S.; Frescas, S.; Nichols, D. E. *Synthetic Comm.* **1995,** *25*, 2775. (b) Capdevielle, P.; Maumy, M. *Tetrahedron Lett.* **1993,** *34*, 1007. (c) Keegstra, M. A.; Peters, T. H.; Brandsma, L. *Tetrahedron* **1992**, *48*, 3633. (d) Yeager, G. W.; Schissel, D. N. *Synthesis* **1991,** 63. (e) Aalten, H. L.; Van Koten, G.; Grove, D. M.; Kuilman, T.; Piekstra, O. G.; Hulshof, L. A.; Sheldon, R. A. *Tetrahedron* **1989,** *45*, 5565. Pentavalent organobismuth reagents have been used in the synthesis of aryl ethers in the presence and absence of Cu salts, see: (d) Barton, D. H. R.; Finet, J.-P.; Khamsi. J.; Pichon, C. *Tetrahedron Lett.* **1986,** *27,* 3619. (e) Barton, D. H. R.; Finet, J.-P.; Motherwell, W. B.; Pichon, C. *J. Chem. Soc., Perkin Trans. I* **1987,** 251.

(4) A variety of electron-deficient transition metal complexes have been used as activators for the synthesis of aryl ethers from the reaction of aryl fluorides and aryl chlorides with alcohols see: (a) Pearson, A. J.; Bruhn, P. R.; Gouzoules, F.; Lee, S-H. *J. Chem. Soc., Chem. Commun.* **1989,** 659. Pearson, A. J.; Gelormini, A. M. *J. Org. Chem.* **1994**, *59*, 4561. (c) Moriarty, R. M.; Ku, Y-Y.; Gill, U. S. *Organometallics* **1988,** 7, 660. (d) Baldoli, C.; Buttero, P. D.; Maiorana, S.; Papagni, A. *J. Chem. Soc., Chem. Commun.* **1985**, 1181. (e) Percec, V.; Okita, S. *J. Polym. Sci. Part A: Polym. Chem.* **1993**, *31*, 923.

(5) Palucki, M.; Wolfe, J. P.; Buchwald, S. L. *J. Am. Chem. Soc.* **1996**, *118*, 10333.

(6) Mann, G.; Hartwig, J. F. *J. Am. Chem. Soc.* **1996**, *118*, 13109.

(7) For examples of nickel-catalyzed synthesis of aryl ethers, see: (a) Cramer, R.; Coulson, D. R. *J Org. Chem.* **1975**, *40*, 2267. (b) Cristau, H.-J.; Desmurs, J.-R. *Ind. Chem. Libr.* **1995**, *7*, 249.

(8) It has been reported that treatment of *trans*-[PdBr(C6H5)(PPh3)2] with a solution of NaOMe in toluene at 35 °C afforded benzene (80% yield), HCHO (20% yield) and anisole (trace) see: Yoshida, T.; Okano, T.; Otsuka, S. *J. Chem. Soc., Dalton Trans.* **1976**, 993.

(9) Either enantiomer of Tol-BINAP can be used as can BINAP. However BINAP is approximately 2.6 times more expensive (Strem Chemicals) than Tol-BINAP.

(10) Reactions performed at 100 °C using Pd(OAc)2 as precatalyst afforded an aryl ether to benzonitrile ratio of 6:1, whereas the benzonitrile side-product was not observed using Pd2(dba)3 as precatalyst.

(11) Since only one product was detected by GC and TLC analyses, and the stereochemistry of the carbinol carbon was preserved in the Pd-catalyzed intramolecular coupling reaction (see reference 5), it is assumed that the stereochemistry of (1*R*, 2*S*, 5*R*)-(-)-menthol is preserved during the course of the reaction.

(12) The following is a representative procedure: An-oven dried 25 mL Schlenk flask was charged with NaH (60% dispersion in mineral oil, 40 mg, 1.00 mmol), 2-propanol (46 µL, 0.60 mmol) and toluene (2 mL) under an argon atmosphere. The mixture was heated at 50 °C for 15 min, cooled to room temperature, and 4-bromobenzonitrile (91 mg, 0.50 mmol), Pd2(dba)3 (6.9 mg, 0.0075 mmol), (*R*)-(+)-2,2'-(di-*p*-tolylphosphino)-1,1'-binaphthyl (Tol-BINAP) (12.2 mg, 0.018 mmol), and 1 mL of toluene were added. The mixture was heated at 50 °C under argon for 22 h and then cooled to room temperature. Water (50 mL) and diethyl ether (50 mL) were added, and the aqueous layer separated and extracted with diethyl ether (50 mL). The organic layers were combined, washed with brine (50 mL), and dried over anhydrous Mg2SO4. The drying agent was removed by filtration and the mother liquor concentrated *in vacuo.* The crude product was purified by flash chromatography on silica gel (19:1 hexanes: ethyl acetate) to afford 4-isopropoxybenzonitrile (65 mg. 80% yield) as a colorless oil.

(13) Zask, A.; Helquist, P. *J. Org. Chem.* **1978,** *43*, 1619.

(14) (a) With Pd(OAc)2 as precatalyst a ratio of 9.2:1 aryl ether:*t*-butylbenzene was obtained; using Pd2(dba)3 this ratio was 4:1 (GC analyses-uncorrected for response factors). This trend is the opposite of that observed in the Pd-catalyzed coupling of 4-bromobenzonitrile with 2-propanol. (b) No meta product, as would be expected from benzyne formation, was observed in this process. (c) Both *t*-butylbenzene and 4,4'-di-*t*-butylbiphenyl were side products of the palladium-catalyzed reaction of 4-*t*-butylbromobenzene with NaO*t*Bu. We are uncertain about the mechanism of formation of *t*-butylbenzene in this reaction. (d) In contrast to the other substrates examined (Table 1), use of *t*-BuOH and NaH in place of NaO*t*Bu afforded large amounts of arene side products and only traces of the desired aryl ether product.

(15) GC analysis of the crude reaction mixture of 9-bromoanthracene with tetrahydro-4*H*-pyran-4-ol gave a 10:1 ratio of aryl ether to anthracene, whereas, GC analysis of the crude reaction mixture of 9-bromoanthracene with cyclopentanol gave a 2.4:1 ratio of aryl ether to anthracene. Note: these ratios are not corrected for response factors.

(16) (a) Wolfe, J. P.; Wagaw, S.; Buchwald, S. L. *J. Am. Chem. Soc.* **1996,** *118*, 7215. (b) Hartwig, J. F.; Richards, S.; Barañano, D.; Paul, F *J. Am. Chem. Soc.* **1996,** *18*, 3626. (c) Driver, M.S.; Hartwig, J. F. *J. Am. Chem.Soc.* **1996,** *118*, 7217. (d) Widenhoefer, R. A.; Buchwald, S. L *Organometallics* **1996.** *15*, 2755. (e) Louie, J.; Paul, F.;

Hartwig, J. F. *Organometallics* **1996,** *15*, 2794. (f) Paul, F.; Patt, J.; Hartwig. J. F *Organometallics* **1995,** *14*, 3030.

(17) Direct reductive elimination from [(*R*)-Tol-BINAP]Pd(*p*-C6H4CN)(OCH2CMe3) to afford the aryl ether product in 84% yield has been recently demonstrated in this laboratory.

(18) (a) Stille, J. K. *The Chemistry of the Metal-Carbon Bond*, Vol 2, Hartley, F. R.; Patai, S. Eds., Wiley, New York, 1985, 625. (b) Gillie, A.; Stille, J. K. *J. Am. Chem. Soc.* **1980**, *102*, 4933. (c) The steric effect of ligands on the rate of a reductive-elimination process was first reported by Jones: Jones, W. D.; Kuykendall, V. L. *Inorg. Chem.* **1991**, *30*, 2615.

(19) Nucleophilic substitution reaction conditions typically require 4.0 equivalents of alkoxide relative to aryl bromide at 80-120 °C. See reference 2.

(20) Uncatalyzed reactions performed in DMF that was not strictly anhydrous did not afford aryl ether products.

(21) (a) Cram, D. J.; Rickbom, R.; Knox, G. R. *J. Am. Chem. Soc.* **1960,** *82*, 6412. (b) Hales, R. H.; Bradshaw, J. S.; Pratt, D. R.; *J. Org. Chem.* **1971,** *36*, 314.

(22) Mauleon, D.; Granados, R.; Minguillon *J. Org. Chem. Soc.* **1983,** *48*, 3105.

(23) Aldrich Chemical Company

## Claims

1. A method of preparing an aryl ether, comprising: reacting an alcohol with an aromatic compound comprising an activated substituent, X, in an aromatic hydrocarbon solvent, in the presence of a base and a catalyst selected from the group consisting of complexes of nickel, palladium, and platinum; wherein X is a moiety whose conjugate acid , HX, has a pKa of less than 5.0.

2. A method of preparing an aryl ether, comprising: reacting an alkoxide salt with an aromatic compound comprising an activated substituent, X, in an aromatic hydrocarbon solvent, in the presence of a catalyst selected from the group consisting complexes of nickel, palladium, platinum; wherein X is a moiety whose conjugate acid , HX, has a pKa of less than 5.0.

3. The method of claim 1, wherein the catalyst is present in an amount in the range of 0.0001 to 20 mol% with respect to the alcohol or aromatic compound.

4. The method of claim 1, wherein the catalyst is present in an amount in the range of 0.05 to 5 mol% with respect to the alcohol or aromatic compound.

5. The method of claim 1, wherein the catalyst is present in an amount in the range of 1 to 3 mol% with respect to the alcohol or aromatic compound.

6. The method of claim 2, wherein the catalyst is present in the amount in the range of 0.0001 to 20 mol% with respect to the alkoxide salt or aromatic compound.

7. The method of claim 2, wherein the catalyst is present in an amount in the range of 0.05 to 5 mol% with respect to the alkoxide salt or aromatic compound.

8. The method of claim 2, wherein the catalyst is present in an amount in the range of 1 to 3 mol% with respect to the alkoxide salt or aromatic compound.

9. The method of claim 2, wherein the reaction occurs in the presence of a base.

10. The method of claim 1 or 2, wherein the reaction is carried out at a temperature in the range of 50°C to 120°C.

11. The method of claim 1 or 2, wherein the reaction is carried out at a temperature in the range of 65°C to 100°C.

12. The method of claim 1 or 2, wherein the aromatic hydrocarbon solvent is toluene.

13. The method of claim 12, wherein the reaction is carried out at a temperature in the range of 50°C to 120°C.

14. The method of claim 12, wherein the reaction is carried out at a temperature in the range of 65°C to 100°C.

15. The method of claim 1, 2, 7, 12, or 13, wherein the catalyst is a palladium complex.

16. The method of claim 15, wherein the palladium comprised by the catalyst is derived from tris(disbenzylideneacetone)dipalladium, palladium acetate, or bis(disbenzylideneacetone)palladium.

**Patentansprüche**

1. Verfahren zum Herstellen eines Arylethers, wobei ein Alkohol in einem aromatischen Kohlenwasserstofflösungsmittel mit einer aromatischen Verbindung umgesetzt wird, die einen aktivierten Substituenten X enthält, und die Umsetzung in Gegenwart einer Base und eines Katalysators erfolgt, der aus Nickel-, Palladium- und Platinkomplexen ausgewählt ist, wobei X ein Rest ist, dessen konjugierte Säure HX einen $pK_a$-Wert von unter 5,0 aufweist.

2. Verfahren zum Herstellen eines Arylethers, wobei ein Alkoxidsalz in einem aromatischen Kohlenwasserstofflösungsmittel mit einer aromatischen Verbindung umgesetzt wird, die einen aktivierten Substituenten X enthält, und die Umsetzung in Gegenwart eines Katalysators erfolgt, der aus Nickel-, Palladium- und Platinkomplexen ausgewählt ist, wobei X ein Rest ist, dessen konjugierte Säure HX einen $pK_a$-Wert von unter 5,0 aufweist.

3. Verfahren nach Anspruch 1, worin der Katalysator in einer Menge im Bereich von 0,0001 bis 20 Mol% bezüglich des Alkohols oder der aromatischen Verbindung vorliegt.

4. Verfahren nach Anspruch 1, worin der Katalysator in einer Menge im Bereich von 0,05 bis 5 Mol% bezüglich des Alkohols oder der aromatischen Verbindung vorliegt.

5. Verfahren nach Anspruch 1, worin der Katalysator in einer Menge im Bereich von 1 bis 3 Mol% bezüglich des Alkohols oder der aromatischen Verbindung vorliegt.

6. Verfahren nach Anspruch 2, worin der Katalysator in einer Menge im Bereich von 0,0001 bis 20 Mol% bezüglich des Alkoxidsalzes oder der aromatischen Verbindung vorliegt.

7. Verfahren nach Anspruch 2, worin der Katalysator in einer Menge im Bereich von 0,05 bis 5 Mol% bezüglich des Alkoxidsalzes oder der aromatischen Verbindung vorliegt.

8. Verfahren nach Anspruch 2, worin der Katalysator in einer Menge im Bereich von 1 bis 3 Mol% bezüglich des Alkoxidsalzes oder der aromatischen Verbindung vorliegt.

9. Verfahren nach Anspruch 2, worin die Reaktion in Gegenwart einer Base abläuft.

10. Verfahren nach Anspruch 1 oder 2, worin die Reaktion bei einer Temperatur im Bereich von 50 bis 120 °C durchgeführt wird.

11. Verfahren nach Anspruch 1 oder 2, worin die Reaktion bei einer Temperatur im Bereich von 65 bis 100 °C durchgeführt wird.

12. Verfahren nach Anspruch 1 oder 2, worin das aromatische Kohlenwasserstofflösungsmittel Toluol ist.

13. Verfahren nach Anspruch 12, worin die Reaktion bei einer Temperatur im Bereich von 50 bis 120 °C durchgeführt wird.

14. Verfahren nach Anspruch 12, worin die Reaktion bei einer Temperatur im Bereich von 65 bis 100 °C durchgeführt wird.

**15.** Verfahren nach Anspruch 1, 2, 7, 12 oder 13, worin der Katalysator ein Palladiumkomplex ist.

**16.** Verfahren nach Anspruch 15, worin das in dem Katalysator enthaltene Palladium von Tris(dibenzylidenaceton)-dipalladium, Palladiumacetat oder Bis(dibenzylidenaceton) -palladium abgeleitet ist.

**Revendications**

**1.** Procédé destiné à la préparation d'un éther d'aryle, comprenant : la réaction d'un alcool avec un composé aromatique comprenant un substituant activé, X, dans un solvant d'hydrocarbure aromatique, en présence d'une base et d'un catalyseur sélectionné parmi le groupe consistant en des complexes de nickel, de palladium et de platine ; dans lequel X est un groupe caractéristique dont l'acide conjugué, HX, a un pKa inférieur à 5,0.

**2.** Procédé destiné à la préparation d'un éther d'aryle, comprenant : la réaction d'un sel d'alcoxyde avec un composé aromatique comprenant un substituant activé, X, dans un solvant d'hydrocarbure aromatique, en présence d'un catalyseur sélectionné parmi le groupe consistant en des complexes de nickel, de palladium et de platine ; dans lequel X est un groupe caractéristique dont l'acide conjugué, HX, a un pKa inférieur à 5,0.

**3.** Procédé selon la revendication 1, dans lequel le catalyseur est présent dans des proportions comprises entre 0,0001 et 20 % en mole par rapport à l'alcool ou au composé aromatique.

**4.** Procédé selon la revendication 1, dans lequel le catalyseur est présent dans des proportions comprises entre 0,05 et 5 % en mole par rapport à l'alcool ou au composé aromatique.

**5.** Procédé selon la revendication 1, dans lequel le catalyseur est présent dans des proportions comprises entre 1 et 3 % en mole par rapport à l'alcool ou au composé aromatique.

**6.** Procédé selon la revendication 2, dans lequel le catalyseur est présent dans des proportions comprises entre 0,0001 et 20 % en mole par rapport au sel d'alcoxyde ou au composé aromatique.

**7.** Procédé selon la revendication 2, dans lequel le catalyseur est présent dans des proportions comprises entre 0,05 et 5 % en mole par rapport au sel d'alcoxyde ou au composé aromatique.

**8.** Procédé selon la revendication 2, dans lequel le catalyseur est présent dans des proportions comprises entre 1 et 3 % en mole par rapport au sel d'alcoxyde ou au composé aromatique.

**9.** Procédé selon la revendication 2, dans lequel la réaction se produit en présence d'une base.

**10.** Procédé selon la revendication 1 ou 2, dans lequel la réaction se déroule à une température comprise entre 50°C et 120°C.

**11.** Procédé selon la revendication 1 ou 2, dans lequel la réaction se déroule à une température comprise entre 65°C et 100°C.

**12.** Procédé selon la revendication 1 ou 2, dans lequel le solvant aromatique est le toluène.

**13.** Procédé selon la revendication 12, dans lequel la réaction se déroule à une température comprise entre 50°C et 120°C.

**14.** Procédé selon la revendication 12, dans lequel la réaction se déroule à une température comprise entre 65°C et 100°C.

**15.** Procédé selon l'une quelconque des revendications 1, 2, 7, 12 ou 13, dans lequel le catalyseur est un complexe de palladium.

**16.** Procédé selon la revendication 15, dans lequel le palladium du catalyseur dérive du tris(disbenzylidène-acétone) dipalladium, de l'acétate de palladium ou du bis(disbenzylidène-acétone)palladium.

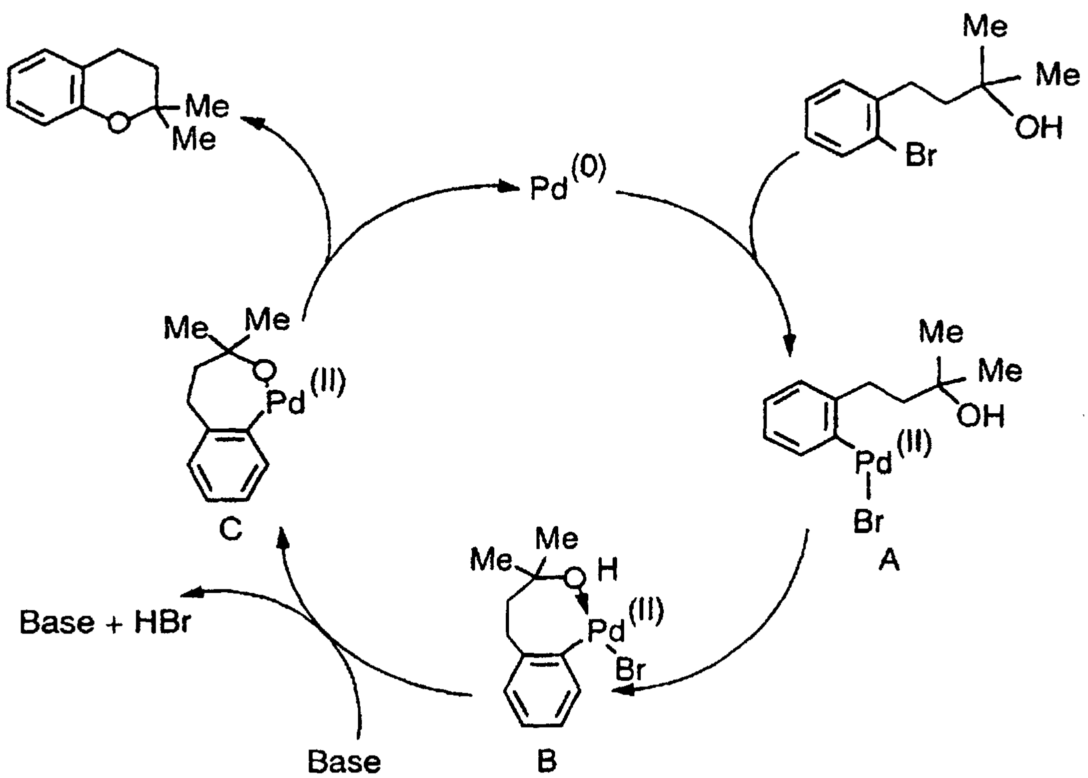

FIG. 1

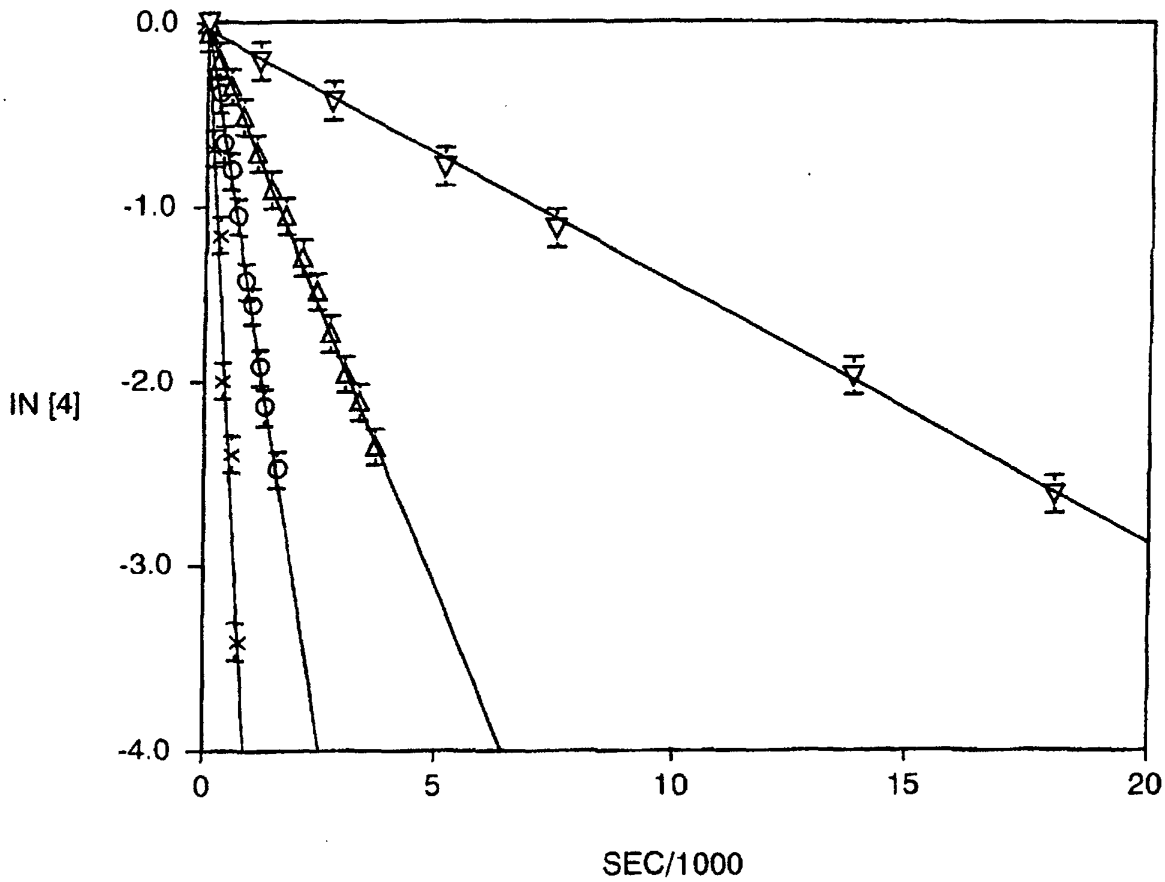


FIG. 2

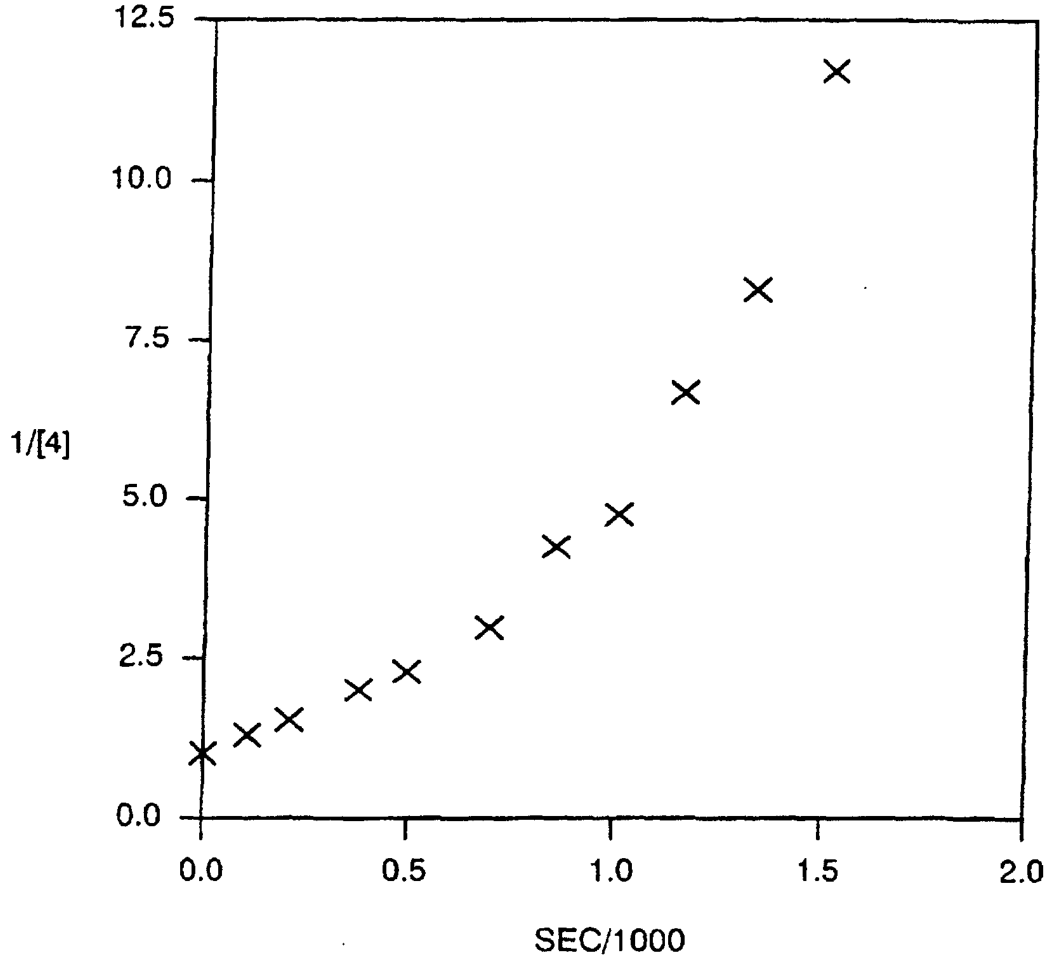


FIG. 3

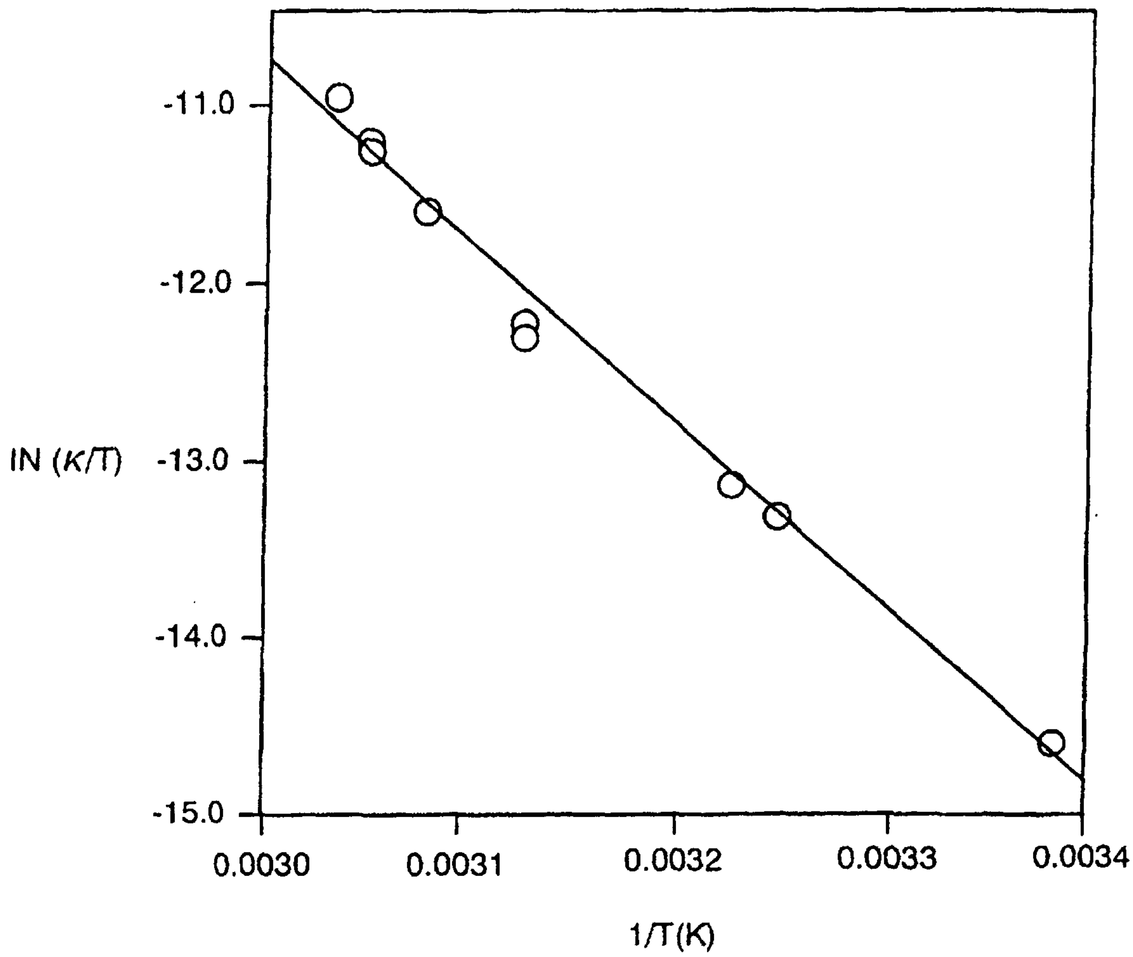


FIG. 4

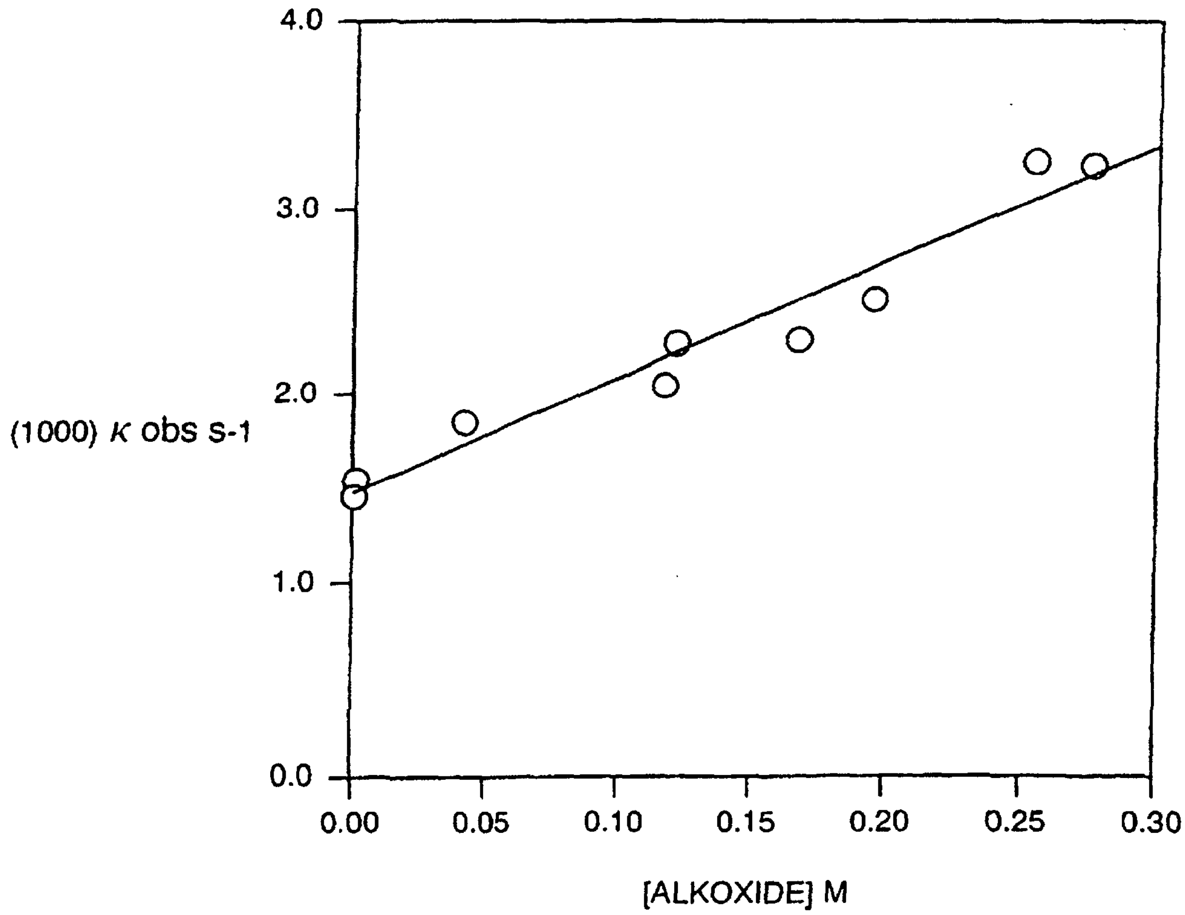
4.0
3.0
2.0
1.0
0.0
(1000) κ obs s-1
0.00
0.05
0.10
0.15
0.20
0.25
0.30
[ALKOXIDE] M

FIG. 5

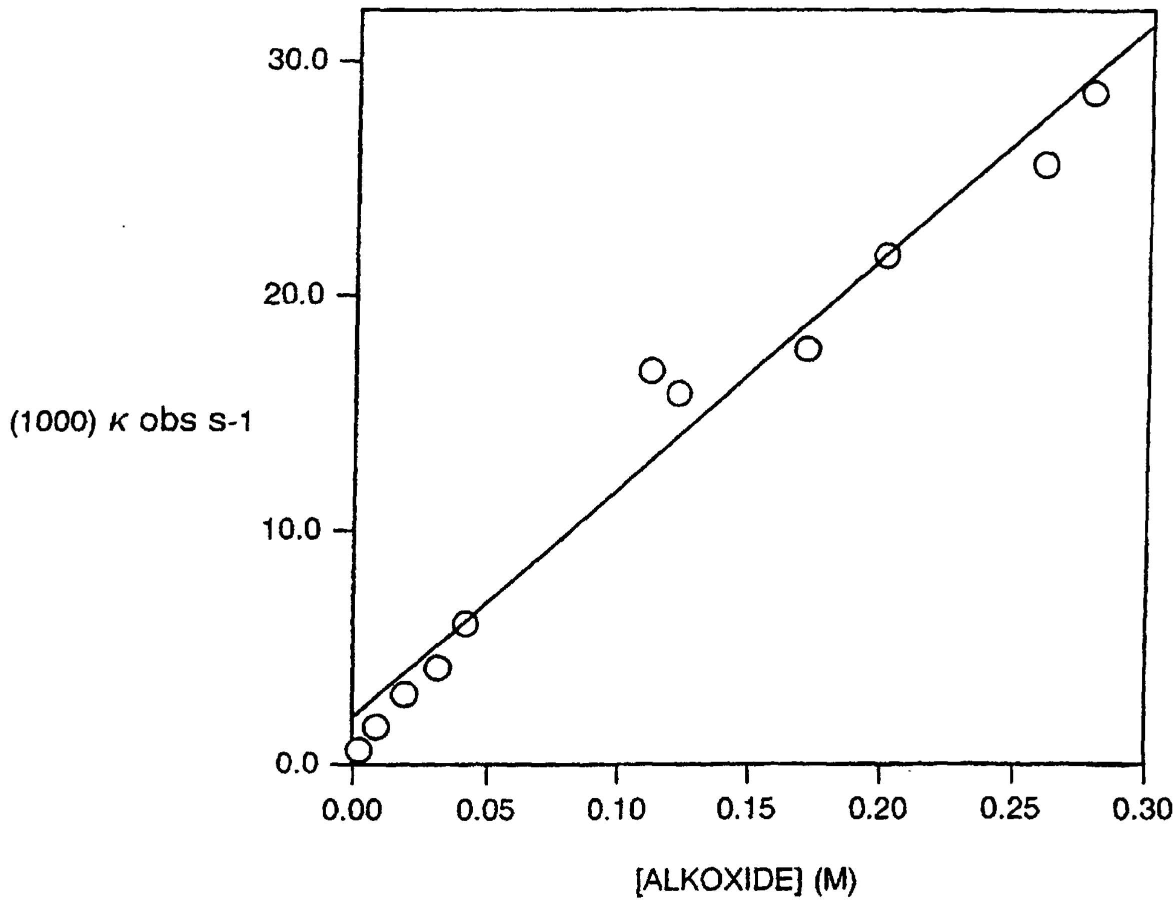
30.0
20.0
10.0
0.0
(1000) κ obs s-1
0.00
0.05
0.10
0.15
0.20
0.25
0.30
[ALKOXIDE] (M)

FIG. 6